# FASCICULE DE BREVET EUROPEEN

(11) **EP 1 701 974 B1**
(45) Date de publication et mention de la délivrance du brevet: **26.03.2008**
(21) Numéro de dépôt: 04816363.8
(22) Date de dépôt: 08.12.2004
(51) Int. Cl.: C07K 5/06, A61K 38/05

(54) **NOUVEAUX DERIVES DU 2-HYDROXYTETRAHYDROFURANNE ET LEUR APPLICATION A TITRE DE MEDICAMENTS**
2-HYDROXYTETRAHYDROFURANDERIVATE UND IHRE VERWENDUNG ALS ARZNEIMITTEL
NOVEL 2-HYDROXYTETRAHYDROFURANE DERIVATIVES AND USE THEREOF AS MEDICAMENTS

(30) Priorité: 09.12.2003 FR 0314368
(43) Date de publication de la demande: 20.09.2006
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUVIN, Serge, F-91120 Palaiseau (FR); CHABRIER DE LASSAUNIERE, Pierre, Etienne, F-75016 Paris (FR)
(74) Mandataire: Bourgouin, André
(86) Numéro de dépôt international: PCT/FR2004/003147
(87) Numéro de publication internationale: WO 2005/056551

(56) Documents cités:
- WO-A-01/32654

## Description

La présente invention concerne de nouveaux dérivés du 2-hydroxytétrahydrofuranne présentant une activité inhibitrice des calpaïnes et / ou une activité piégeuse des formes réactives de l'oxygène (ROS pour "reactive oxygen species "). L'invention concerne également leurs méthodes de préparation, les préparations pharmaceutiques les contenant et leur utilisation à des fins thérapeutiques, en particulier en tant qu'inhibiteurs de calpaïnes et piégeurs de formes réactives de l'oxygène de manière sélective ou non.

Compte tenu du rôle potentiel des calpaïnes et des ROS en physiopathologie, les nouveaux dérivés selon l'invention peuvent produire des effets bénéfiques ou favorables dans le traitement de pathologies où ces enzymes et / ou ces espèces radicalaires sont impliquées, et notamment :
- les maladies inflammatoires et immunologiques comme par exemple l'arthrite rhumatoïde, les pancréatites, la sclérose en plaques, les inflammations du système gastro-intestinal (colite ulcérative ou non, maladie de Crohn),
- les maladies cardio-vasculaires et cérébro-vasculaires comprenant par exemple l'hypertension artérielle, le choc septique, les infarctus cardiaques ou cérébraux d'origine ischémique ou hémorragique, les ischémies ainsi que les troubles liés à l'agrégation plaquettaire,
- les troubles du système nerveux central ou périphérique comme par exemple les maladies neurodégénératives où l'on peut notamment citer les traumatismes cérébraux ou de la moelle épinière, l'hémorragie sub arachnoïde, l'épilepsie, le vieillissement, les démences séniles, y compris la maladie d'Alzheimer, la chorée de Huntington, la maladie de Parkinson, les neuropathies périphériques,
- la perte d'audition,
- l'ostéoporose,
- les dystrophies musculaires,
- les maladies prolifératives comme par exemple l'athérosclérose ou la resténose,
- la cataracte,
- les transplantations d'organes,
- les maladies auto-immunes et virales comme par exemple le lupus, le sida, les infections parasitaires et virales, le diabète et ses complications, la sclérose en plaques,
- le cancer,
- toutes les pathologies caractérisées par une production excessive des ROS et / ou une activation des calpaïnes.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des calpaïnes (Trends Pharmacol. Sci. (1994) 15, 412419 ; Drug News Perspect (1999) 12, 73-82). A titre d'exemple, les lésions cérébrales associées à l'infarctus cérébral ou au traumatisme crânien expérimental sont réduites par des agents antioxydants (Acta. Physiol. Scand. (1994) 152, 349-350 ; J. Cereb. Blood Flow Metabol. (1995) 15, 948-952 ; J Pharmacol Exp Ther (1997) 2, 895-904) ainsi que par des inhibiteurs de calpaïnes (Proc Natl Acad Sci U S A (1996) 93, 3428-33 ; Stroke, (1998) 29, 152-158; Stroke (1994) 25, 2265-2270).

La demanderesse avait déjà décrit dans la demande de brevet PCT WO 01/32654 des composés hétérocycliques présentant à la fois une activité inhibitrice des calpaïnes et une activité de piégeage des formes réactives de l'oxygène.

Lesdits composés hétérocycliques de ladite demande de brevet répondent à la formule générale (A1) 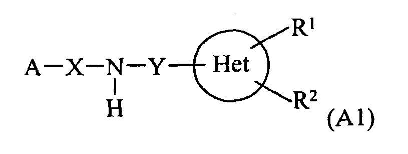 dans laquelle
R¹ représente un atome d'hydrogène, un radical -OR³, -SR³, oxo ou un acétal cyclique, dans lequel R³ représente un atome d'hydrogène, un radical alkyle, arylalkyle, hétérocycloalkylcarbonyle, alkylcarbonyle, arylcarbonyle ou aralkylcarbonyle, dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement substitué,
R² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
   dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle ;
A représente notamment un radical phénothiazinyle éventuellement substitué ;
X représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Y représente -(CH₂)ₚ- , -C(R⁵³R⁵⁴)-(CH₂)ₚ-, -C(R⁵³R⁵⁴)-CO- ;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Het représente un hétérocycle,
ainsi que les sels d'addition avec les acides minéraux et organiques ou avec les bases minérales et organiques desdits composés de formule générale (A1),
à l'exclusion des composés de formule (A1) dans laquelle lorsque Het représente tétrahydrofuranne ou tétrahydropyranne, R¹ le radical OR³ avec R³ représentant un atome d'hydrogène, un radical alkyle, arylalkyle, hétérocycloalkylcarbonyle dont le radical hétérocycloalkyle est branché par un atome de carbone, alkylcarbonyle, arylcarbonyle ou aralkylcarbonyle, R² un hydrogène et Y le radical -(CH₂)ₚ- avec p = 0, alors X ne représente pas -CO-N(R⁴⁵)-C(R⁴⁶R⁴⁷)-CO- avec R⁴⁵ = R⁴⁶ = H.

La demanderesse a à présent constaté de façon surprenante que les composés de formule générale **(I)** décrits ci-après présentent à la fois une activité inhibitrice des calpaïnes et une activité de piégeage des formes réactives de l'oxygène tout en possédant des propriétés améliorées en termes de pénétration cellulaire.

La présente invention a donc pour objet des composés de formule générale **(I)** 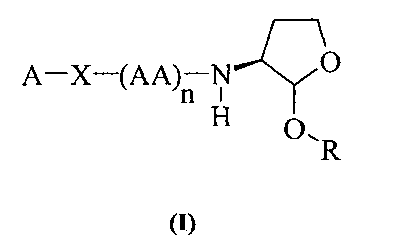 dans laquelle :
A représente le radical 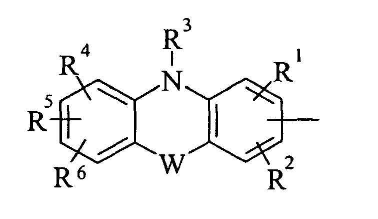 dans lequel
   R¹, R², R⁴, R⁵ et R⁶ représentent, indépendamment, un atome d'hydrogène, un atome halogène, le groupe OH, un radical alkyle, alkoxy, cyano, nitro ou NR⁷R⁸,
   R⁷ et R⁸ représentent, indépendamment, un atome d'hydrogène, un radical alkyle ou un groupe -COR⁹,
   R⁹ représente un atome d'hydrogène, un radical alkyle ou alkoxy,
   R³ représente un atome d'hydrogène, un radical alkyle ou un groupe -COR¹⁰,
   R¹⁰ représente un atome d'hydrogène ou un radical alkyle ou alkoxy, et
   W représente une liaison ou un radical -CH₂-CH₂-, -CH=CH-, -O-, -S- ou -NR¹¹- dans lequel R¹¹ représente un atome d'hydrogène ou un radical alkyle ;
X représente -CO-, -Y-CO-, -O-Y-CO- ou -NR¹²-Y-CO-,
Y représente un radical alkylène ou haloalkylène,
R¹² représente un atome d'hydrogène, un radical alkyle ou un groupe -COR¹³,
R¹³ représente un atome d'hydrogène, un radical alkyle, haloalkyle ou alkoxy,
AA représente, à chaque fois qu'il intervient, un aminoacide naturel, un aminoacide naturel dont la chaîne latérale qui porte une fonction chimique réactive (telle qu'acide carboxylique, amine, alcool ou thiol) est protégée sous forme d'ester d'alkyle ou d'aralkyle (pour les fonctions acide), de carbamate d'alkyle, d'aralkyle ou bien de carboxamide d'alkyle ou d'aralkyle (pour les fonctions amine), sous forme d'éther d'alkyle ou d'aralkyle ou de thioéther d'alkyle ou d'aralkyle ou bien sous forme d'ester d'alkyle ou d'aralkyle (pour les fonctions alcool et thiol) ou enfin un aminoacide de formule générale -NR¹⁴-(CH₂)ₚ CR¹⁵R¹⁶-CO- dans laquelle p représente 0 ou 1, R¹⁴ représente un atome d'hydrogène ou un radical alkyle, R¹⁵ représente un atome d'hydrogène ou un radical alkyle et R¹⁶ un atome d'hydrogène, un radical alkyle, haloalkyle, phényle, cycloalkyle, cycloalkylalkyle ou alkényle,
   ou bien R¹⁵ et R¹⁶ formant avec l'atome de carbone auquel ils sont attachés un carbocycle saturé de 3 à 7 atomes de carbone (et de préférence de 3 à 6 atomes de carbone),
   un groupe -(AA)₂- pouvant aussi représenter un carbapeptide de formule générale -NR¹⁷-(CH₂)₃-CH(R¹⁸)-CO- dans laquelle R¹⁷ représente un atome d'hydrogène ou un radical alkyle et R¹⁸ représente un atome d'hydrogène ou un radical alkyle ;
n représente 2 ou 3 ; et enfin
R représente un atome d'hydrogène ou un radical alkyle ou -CO-R¹⁹ dans lequel R¹⁹ représente un radical alkyle (et en particulier méthyle) ;
ou les sels de tels composés.

Par alkyle ou alkylène, lorsqu'il n'est pas donné plus de précision, on entend un radical alkyle ou alkylène linéaire ou ramifié comptant de 1 à 12 atomes de carbone, et de préférence de 1 à 6 atomes de carbone. Par haloalkyle ou haloalkylène, on entend un radical alkyle ou alkylène dont l'un au moins des atomes d'hydrogène est substitué par un atome halogène. Par alkényle, lorsqu'il n'est pas donné plus de précision, on entend un radical alkényle linéaire ou ramifié comptant de 2 à 12 atomes de carbone, et de préférence de 2 à 6 atomes de carbone. Par cycloalkyle, lorsqu'il n'est pas donné plus de précision, on entend un radical cycloalkyle comptant de 3 à 7 atomes de carbone. Par alkoxy, lorsqu'il n'est pas donné plus de précision, on entend un radical alkoxy dont la chaîne carbonée est linéaire ou ramifiée et compte de 1 à 6 atomes de carbone. Par aryle, lorsqu'il n'est pas donné plus de précision, on entend un radical aryle carbocyclique. Par aryle carbocyclique, on entend un radical aryle carbocyclique comptant de 1 à 3 cycles fusionnés. Enfin, par atome halogène, on entend un atome choisi parmi les atomes de fluor, de chlore, de brome et d'iode.

Par les radicaux aralkyle et cycloalkylalkyle, on entend respectivement les radicaux aralkyle et cycloalkylalkyle dont les radicaux alkyle, aryle et cycloalkyle qui les composent ont les significations indiquées précédemment.

Par acide aminé naturel, on entend la valine (Val), la leucine (Leu), l'isoleucine (Ile), la méthionine (Met), la phénylalanine (Phe), l'asparagine (Asn), l'acide glutamique (Glu), la glutamine (Gln), l'histidine (His), la lysine (Lys), l'arginine (Arg), l'acide aspartique (Asp), la glycine (Gly), l'alanine (Ala), la sérine (Ser), la thréonine (Thr), la tyrosine (Tyr), le tryptophane (Trp), la cystéine (Cys) ou la proline (Pro).

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par cycloalkyle comptant de 3 à 7 atomes de carbone, on entend en particulier un radical cyclohexyle. Par aryle carbocyclique, on entend notamment les radicaux phényle, naphtyle et phénantryle, de préférence les radicaux phényle et naphtyle et plus préférentiellement le radical phényle. Par haloalkyle, on entend en particulier le radical -CF₃. Enfin, par haloalkylène, on entend notamment le radical -CF₂-.

Des exemples de fonctions protégées portées par des chaînes latérales d'aminoacides naturels comprennent notamment :
- des fonctions acide protégées sous forme d'ester de méthyle, d'éthyle, de *tert*-butyle ou de benzyle ;
- des fonctions amine protégées sous forme de carbamate de *tert*-butyle ou de benzyle, d'acétamide ;
- des fonctions alcool protégées sous forme d'éther de tert-butyle, de benzyle ou de pyrane ou encore sous forme d'acétyle ; et
- des fonctions thiol protégées sous forme de thioéthers de méthyle ou sous forme de thioesters de méthyle.

De préférence, les composés de l'invention seront tels qu'ils possèderont au moins l'une des caractéristiques suivantes :
❖ R¹, R², R⁴, R⁵ et R⁶ représentent, indépendamment, un atome d'hydrogène, un atome halogène ou un radical alkyle, alkoxy ou NR⁷R⁸;
❖ R³ représente un atome d'hydrogène, un radical méthyle ou un radical -COR⁹ dans lequel R⁹ représente un radical méthyle ou *tert*-butoxy ;
❖ W représente une liaison ou un radical -CH₂-CH₂-, -CH=CH-, -O- ou -S-;
❖ X représente -CO-, -Y-CO- ou -O-Y-CO- ;
❖ -(AA)ₙ- contient des aminoacides choisis indépendamment parmi le groupe constitué par les aminoacides naturels, la 3-méthylvaline, la norvaline, la phénylglycine, la vinylglycine et l'acide 2-aminobutyrique ;
❖ n représente 2 ;
❖ R représente un atome d'hydrogène ou un radical méthyle.

Plus préférentiellement, les composés de l'invention seront tels qu'ils possèderont au moins l'une des caractéristiques suivantes :
❖ R¹, R², R⁴, R⁵ et R⁶ représentent, indépendamment, un atome d'hydrogène ou un radical alkyle ou alkoxy (et, plus préférentiellement encore, R¹, R², R⁴, R⁵ et R⁶ sont tous des atomes d'hydrogène) ;
❖ R³ représente un atome d'hydrogène ou un radical méthyle (et, plus préférentiellement encore, un atome d'hydrogène) ;
❖ W représente -S- ;
❖ X représente -Y-CO- ou -O-Y-CO- ;
❖ -(AA)ₙ- représente un groupe -(AA²)-(AA¹)- tel que AA¹ représente Leu et AA² représente un aminoacide choisi parmi le groupe constitué par les aminoacides naturels, la 3-méthylvaline, la norvaline, la phénylglycine, la vinylglycine et l'acide 2-aminobutyrique (et, plus préférentiellement encore, un groupe -(AA²)-(AA¹)- tel que AA¹ représente Leu et AA² représente un aminoacide choisi parmi le groupe constitué par Leu, Lys, Val, la 3-méthylvaline, la norvaline, la phénylglycine, la vinylglycine et l'acide 2-aminobutyrique) ;
❖ R représente un atome d'hydrogène.

En particulier, l'invention concerne un composé de formule générale **(I)** choisi parmi les composés suivants :
- *N-*(10H-phénothiazin-2-ylcarbonyl)-L-leucyl-L-leucyl-*N¹*-[(3*S*)-2-méthoxytétrahydrofuran-3-yl]-L-leucinamide ;
- *N*-(10*H*-phénothiazin-2-ylcarbonyl)-L-leucyl-L-leucyl-*N¹*-[(3*S*)-2-hydroxytétrahydrofuran-3-yl]-L-leucinamide ;
- *N*-(10H-phénothiazin-2-ylcarbonyl)glycyl-*N¹*-[(3S)-2-méthoxytétrahydrofuran-3-yl]-L-leucinamide ;
- *N*-(10H-phénothiazin-2-ylcarbonyl)leucyl-*N¹*-[(3S)-2-méthoxytétrahydrofuran-3-yl]-L-leucinamide ;
- *N*⁶-[(benzyloxy)carbonyl]-*N²*-(10H-phénothiazin-2-ylcarbonyl)lysyl-*N¹*-[(3S)-2-méthoxytétrahydrofuran-3-yl]-L-leucinamide ;
- 1-(10H-phénothiazin-2-ylcarbonyl)-L-prolyl-*N¹*-[(3S)-2-méthoxytétrahydrofuran-3-yl]-L-leucinamide ;
- *N*-(10H-phénothiazin-2-ylcarbonyl)glycyl-*N¹*-[(3S)-2-hydroxytétrahydrofuran-3-yl]-L-leucinamide ;
- *N*-(10H-phénothiazin-2-ylcarbonyl)leucyl-*N¹*-[(3S)-2-hydroxytétrahydrofuran-3-yl]-L-leucinamide ;
- *N⁶*-[(benzyloxy)carbonyl]-*N*²-(10H-phénothiazin-2-ylcarbonyl)lysyl-*N*¹-[(3S)-2-hydroxytétrahydrofuran-3-yl]-L-leucinamide ;
- 1-(10H-phénothiazin-2-ylcarbonyl)-L-prolyl-*N*¹-[(3S)-2-hydroxytétrahydrofuran-3-yl]-L-leucinamide ;
- *N*-(10H-phénothiazin-2-ylcarbonyl)leucyl-*N*¹-[(3S)-2-(acétyloxy)-tétrahydrofuran-3-yl]-L-leucinamide ;
- *N²*-(10H-phénothiazin-2-ylcarbonyl)lysyl-*N¹*-[(3S)-2-hydroxytétrahydrofuran-3-yl]-L-leucinamide ;
- *N*-(10H-phénothiazin-2-ylacétyl)-L-leucyl-*N¹*-[(3S)-2-méthoxytétrahydrofuran-3-yl]-L-leucinamide ;
- *O*-(tert-butyl)-*N*-(10H-phénothiazin-2-ylacétyl)-L-séryl-*N¹*-[(3S)-2-méthoxytétrahydrofuran-3-yl]-L-leucinamide ;
- *N*-(10H-phénothiazin-2-ylacétyl)-L-alanyl-3-cyclohexyl-*N¹*-[(3S)-2-méthoxytétrahydrofuran-3-yl]-L-alaninamide ;
- *N*-(10H-phénothiazin-2-ylacétyl)-L-leucyl-*N¹*-[(3S)-2-hydroxytétrahydrofuran-3-yl]-L-leucinamide ;
- *O*-(tert-butyl)-*N*-(10H-phénothiazin-2-ylacétyl)-L-séryl-*N¹*-[(3S)-2-hydroxytétrahydrofuran-3-yl]-L-leucinamide ;
- *N*-(10H-phénothiazin-2-ylacétyl)-L-alanyl-3-cyclohexyl-*N*¹-[(3S)-2-hydroxytétrahydrofuran-3-yl]-L-alaninamide ;
- *N*-[3-(10H-phénothiazin-2-yl)propanoyl]-L-leucyl-*N*¹-[(3S)-2-méthoxytétrahydrofuran-3-yl]-L-leucinamide ;
- *N*-[3-(10H-phénothiazin-2-yl)propanoyl]-L-leucyl-*N¹*-[(3S)-2-hydroxytétrahydrofuran-3-yl]-L-leucinamide ;
- *N*-[(10H-phénothiazin-2-yloxy)acétyl]-L-leucyl-*N¹*-[(3S)-2-méthoxytétrahydrofuran-3-yl]-L-leucinamide ;
- *N*-[(10H-phénothiazin-2-yloxy)acétyl]-glycyl-*N¹*-[(3S)-2-méthoxytétrahydrofuran-3-yl]-L-leucinamide ;
- *N*-[(10H-phénothiazin-2-yloxy)acétyl]-L-alanyl-*N*¹-[(3S)-2-méthoxytétrahydrofuran-3-yl]-L-leucinamide ;
- *N*-[(10H-phénothiazin-2-yloxy)acétyl]-L-valyl-*N*¹-[(3S)-2-méthoxytétrahydrofuran-3-yl]-L-leucinarnide ;
- *N*-[(10H-phénothiazin-2-yloxy)acétyl]-β-alanyl-*N*¹-[(3S)-2-méthoxytétrahydrofuran-3-yl]-L-leucinamide ;
- *N*-méthyl-*N*-[(10H-phénothiazin-2-yloxy)acétyl]glycyl-*N*¹-[(3S)-2-méthoxytétrahydrofuran-3-yl]-L-leucinamide
- *N*-[(10H-phénothiazin-2-yloxy)acétyl]-D-valyl-*N*¹-[(3S)-2-méthoxytétrahydrofuran-3-yl]-L-leucinamide ;
- 3-methyl-*N*-[(10H-phénothiazin-2-yloxy)acétyl]-L-valyl-*N¹* -[(3S)-2-méthoxytétrahydrofuran-3-yl]-L-leucinamide ;
- *N¹*-[(3S)-2-méthoxytétrahydrofuran-3-yl]-*N²*-((2S)-2- {[(10H-phénothiazin-2-yloxy)-acétyl]amino}butanoyl)-L-leucinamide ;
- *N*-[(10H-phénothiazin-2-yloxy)acétyl]-L-norvalyl-*N¹*-[(3S)-2-méthoxytétrahydrofuran-3-yl]-L-leucinamide ;
- *N*-[(10H-phénothiazin-2-yloxy)acétyl]-L-séryl-*N¹*-[(3S)-2-méthoxytétrahydrofuran-3-yl]-L-leucinamide ;
- *N-[*(10H-phénothiazin-2-yloxy)acétyl]-L-thréonyl-*N¹*-((3S)-2-méthoxytétrahydrofuran-3-yl]-L-leucinamide ;
- *N*¹-[(3S)-2-méthoxytétrahydrofuran-3-yl]-*N*²-((2S)-2-{[(10H-phénothiazin-2-yloxy)acétyl]amino}-2-phényléthanoyl)-L-leucinamide ;
- *N¹*-[(3S)-2-méthoxytétrahydrofuran-3-yl]-*N²*-((2S)-2-{[(10H-phénothiazin-2-yloxy)acétyl]amino}but-3-enoyl)-L-leucinamide ;
- 2-méthyl-*N*-[(10H-phénothiazin-2-yloxy)acétyl]alanyl-*N¹*-[(3S)-2-méthoxytétrahydrofuran-3-yl)-L-leucinamide ;
- *N*-[(10H-phénothiazin-2-yloxy)acétyl]-glycyl-*N¹*-((3S)-2-méthoxytétrahydrofuran-3-yl]-L-valinamide ;
- *N*-[(10H-phénothiazin-2-yloxy)acétyl]-glycyl-3-cyclohexyl-*N¹*-[(3S)-2-méthoxytétrahydrofuran-3-yl]-L-alaninamide ;
- *N*-[(10H-phénothiazin-2yloxy)acétyl]-glycyl-*N*-[(35)-2-méthoxytétrahydrofuran-3-yl]-L-phénylalaninamide ;
- *N-*[(10H-phénothiazin-2-yloxy)acétyl]glycyl-*N²*-isobutyl-*N*¹-[(3S)-2-méthoxytétrahydrofuran-3-yl]glycinamide ;
- *N*-[(10H-phénothiazin-2-yloxy)acétyl]-L-leucyl-*N*¹-[(3S)-2-hydroxytétraliydrofuran-3-yl]-L-leucinamide ;
- *N*-[(10H-phénothiazin-2-yloxy)acétyl]-glycyl-*N*¹-[(3S)-2-hydroxytétrahydrofuran-3-yl]-L-leucinamide ;
- *N-[*(10H-phénothiazin-2-yloxy)acétyl]-L-alanyl-*N*¹-[(3S)-2-hydroxytétrahydrofuran-3-yl]-L-leucinamide ;
- *N-[*(10H-phénothiazin-2-yloxy)acétyl]-L-valyl-*N*¹-[(3S)-2-hydroxytétrahydrofuran-3-yl]-L-leucinamide ;
- *N*-[(10H-phénothiazin-2-yloxy)acétyl]-β-alanyl-*N*¹-[(3S)-2-hydroxytétrahydrofuran-3-yl]-L-leucinamide ;
- *N*-méthyl-*N*[(10H-phénothiazin-2-yloxy)acétyl]glycyl-*N*¹-[(3S)-2-hydroxytétrahydrofuran-3-yl]-L-leucinamide ;
- *N*-[(10H-phénothiazin-2-yloxy)acétyl]-D-valyl-*N*¹-[(3S)-2-hydroxytétrahydrofuran-3-yl]-L-leucinamide ;
- 3-méthyl-*N-*[(10H-phénothiazin-2-yloxy)acétyl]-L-valyl-*N*¹-[(3S)-2-hydroxytétrahydrofuran-3-yl]-L-leucinamide ;
- *N*¹-[(3S)-2-hydroxytétrahydrofuran-3-yl]-*N*²-((2S)-2-{[(10H-phénothiazin-2-yloxy)acétyl]amino}butanoyl)-L-leucinamide ;
- *N*-[(10H-phénothiazin-2-yloxy)acétyl]-L-norvalyl-*N¹*-[(3S)-2-hydroxytétrahydrofuran-3-yl]-L-leucinamide ;
- *N*-[(10H-phénothiazin-2-yloxy)acétyl]-L-séryl-*N*¹-[(3S)-2-hydroxytétrahydrofuran. 3-yl]-L-leucinamide ;
- *N*-[(10H-phénothiazin-2-yloxy)acétyl]-L-thréonyl-]*N¹*-[(3S)-2-hydroxytétrahydrofuran-3-yl]-L-leucinamide ;
- *N*¹-[(3 S)-2-hydroxytétrahydrofuran-3-yl]-*N*²-((2S)-2- {[(10H-phénothiazin-2-yloxy)acétyl]amino}-2-phényléthanoyl)-L-leucinamide ;
- *N*¹-[(3S)-2-hydroxytétrahydrofuran-3-yl]-*N*²-((2S)-2-{[(10H-phénothiazin-2-yloxy)-acétyl]amino}but-3-énoyl)-L-leucinamide ;
- 2-méthyl-*N*-[(10H-phénothiazin-2-yloxy)acétyl]alanyl-*N*¹-[(3S)-2-hydroxytétrahydrofuran-3-yl]-L-leucinamide ;
- *N*-[(10H-phénothiazin-2-yloxy)acétyl]glycyl-*N¹*-[(3S)-2-hydroxytétrahydrofuran-3-yl]-L-valinamide ;
- *N*-[(10H-phénothiazin-2-yloxy)acétyl]glycyl-3-cyclohexyl-*N¹*-[(3S)-2-hydroxytétrahydrofuran-3-yl]-L-alaninamide ;
- *N*-[(10H-phénothiazin-2-yloxy)acétyl]glycyl-*N*-[(3S)-2-hydroxytétrahydrofuran-3-yl]-L-phénylalaninamide ;
- *N*-[(10H-phénothiazin-2-yloxy)acétyl]glycyl-*N¹*-[(3S)-2-hydroxytétrahydrofuran-3-yl]-*N²*-isobutylglycinamide ;
- *N*-[2-métliyl-2-(10H-phénothiazin-2-yloxy)propanoyl]glycyl-*N¹*-[(3S)-2-méthoxytétrahydrofuran-3-yl]-L-leucinamide ;
- *N*-[2-méthyl-2-(10H-phénothiazin-2-yloxy)propanoyl]glycyl-*N*¹-[(35)-2-hydroxytétrahydrokran-3-yl]-L-leucinamide ;
- *N*-(10,11-dihydro-5H-dibenzo[b,f]azépin-3-ylcarbonyl)-L-leucyl-*N*¹-[(3S)-2-méthoxytétrahydrofuran-3-yl]-L-leucinamide ;
- *N-*(10,11-dihydro-5H-dibenzo[b,f]azépin-3-ylcarbonyl)-L-leucyl-*N¹*-[(3S)-2-hydroxytétrahydrofuran-3-yl]-L-leucinamide ;
- *N*-[(5-acétyl-10,11-dihydro-5H-dibenzo[b,f]azepin-3-yl)carbonyl]-L-leucyl-*N¹*-[(3S)-2-méthoxytétrahydrofuran-3-yl]-L-leucinamide ;
- 2-méthyl-*N*-[(10H-phénothiazin-2-yloxy)acétyl]alanyl-*N¹*-[(3S)-2-hydroxytétrahydrofuran-3-yl]-L-leucinamide ;
ou un sel d'un de ces derniers.

La présente invention a également pour objet, à titre de médicaments, les composés de formule générale **(I)** telle que définie précédemment, ainsi que les sels pharmaceutiquement acceptables de tels composés.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

L'invention concerne également les compositions pharmaceutiques contenant, à titre de principe actif, un composé de formule générale **(I)** telle que définie précédemment, ou un sel pharmaceutiquement acceptable d'un tel composé, avec au moins un excipient pharmaceutiquement acceptable.

Les compositions pharmaceutiques contenant un composé de l'invention peuvent être sous forme d'un solide, par exemple des poudres, des granules, des comprimés, des gélules, des liposomes, des suppositoires ou des patchs. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'invention concerne en outre l'utilisation d'un composé de formule générale **(I)** telle que définie précédemment, ou d'un sel pharmaceutiquement acceptable d'un tel composé, pour préparer un médicament destiné à traiter toutes les pathologies caractérisées par une production excessive des ROS et / ou une activation des calpaïnes, et en particulier les maladies et désordres choisis parmi le groupe constitué par les maladies inflammatoires et immunologiques, les maladies cardio-vasculaires et cérébro-vasculaires, les troubles du système nerveux central ou périphérique, l'ostéoporose, les dystrophies musculaires, les maladies prolifératives, la cataracte, les réactions de rejet suite à des transplantations d'organes et les maladies auto-immunes et virales.

L'administration d'un médicament selon l'invention pourra se faire par voie topique, par voie orale, par voie parentérale, par injection intramusculaire, par injection sous-cutanée, par injection intra-veineuse, etc.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A titre indicatif, la dose d'administration envisagée pour un médicament selon l'invention est comprise entre 0,1 mg et 10 g suivant le type de composé actif utilisé.

Conformément à l'invention, on peut préparer les composés de formule générale **(I)** par les procédés décrits ci-après.

### Préparation des composés de formule générale (I)

Les composés de formule **(I)** selon l'invention peuvent être préparés selon la voie de synthèse représentée dans le schéma 1 ci-dessous (les composés de formule générale **(I)**
dans laquelle R représente un radical alkyle Alk étant appelés les composés de formule générale **(I)**₁, ceux de formule générale **(I)** dans laquelle R représente un atome d'hydrogène étant appelés les composés de formule générale **(I)**₂ et ceux de formule générale **(I)** dans laquelle R représente -COR¹⁹ étant appelés les composés de formule générale **(I)**₃ dans la suite de l'exposé) : 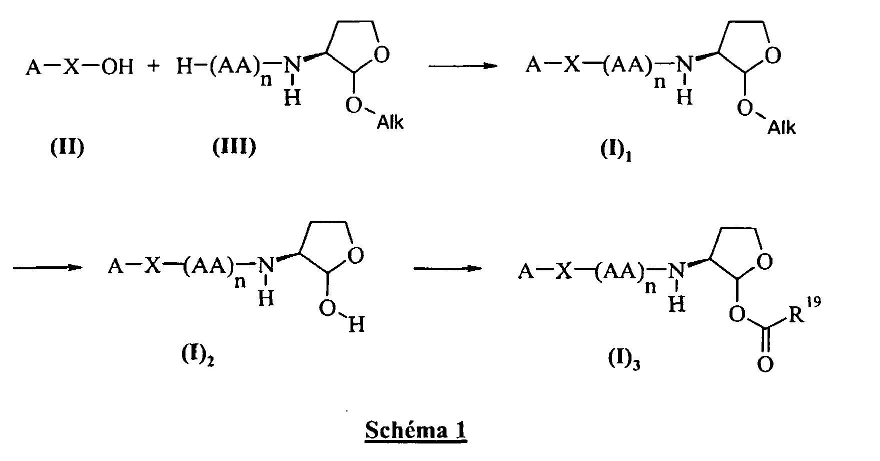 Les composés de formules générales **(I)₁** et **(**I**)**₂ dans lesquelles A, X, AA, n et R sont tels que décrits précédemment sont préparés, schéma 1, par condensation des acides de formule générale **(II)** sur les amines de formule générale **(III),** dans les conditions classiques de la synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) *(*J. Med. Chem. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)) et d'une base telle que, par exemple, la triéthylamine ou la *N,N*-diisopropyléthylamine, pour conduire aux composés de formule générale **(I)**₁. La fonction hémiacétalique des composés de formule générale **(I)₁** peut ensuite être déprotégée à l'aide d'une solution aqueuse environ 2*N* d'un acide minéral, tel que par exemple, HCl ou HBr, en utilisant de l'acétone comme co-solvant. Les dérivés lactols de formule générale **(I)₂** ainsi obtenus peuvent le cas échéant être acylés à l'aide, notamment, d'un anhydride d'acide (R¹⁹CO)₂O (par exemple l'anhydride acétique) en présence d'un agent d'acylation tel que la N,N-diméthyl-4-pyridinamine pour conduire aux composés de formule générale **(I)₃.**

### Préparation des intermédiaires de formule générale (II):

Les acides carboxyliques de formule générale **(II)** non commerciaux dans lesquels A, W, X, Y, R¹, R², R³, R⁴, R⁵ et R⁶ sont tels que décrits ci-dessus, sont accessibles selon différentes voies synthétiques détaillées ci-après.

### Lorsque X =-O-Y-CO-:

Dans ce cas, une voie de synthèse comme celle représentée dans le schéma 2 ci-après peut être utilisée. 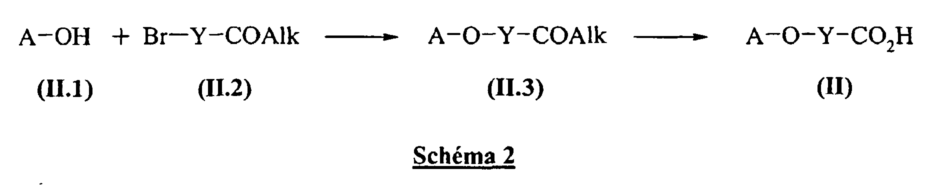 Selon cette voie de synthèse, les acides de formule générale **(II)** dans lesquels X représente -O-Y-CO-, schéma 2, peuvent être préparés à partir, par exemple, d'hydroxyphénothiazines (J. Med. Chem. (1992), 35(4), 716-24) ou d'hydroxycarbazoles (J. Chem. Soc (1955), 3475-3477 ; J. Med. Chem. (1964), 7, 158-161) de formule générale **(II.1).** La condensation sur des halogénoesters commerciaux de formule générale **(II.2)** est effectuée en présence d'une base telle que, par exemple K₂CO₃ ou Cs₂CO₃, en chauffant dans un solvant polaire comme, par exemple, le THF ou le DMF, pendant au moins 5 heures. Les esters de formule générale **(II.3)** intermédiairement obtenus sont ensuite déprotégés (en milieu acide dans le cas des esters de *tert*-butyle ou bien par saponification pour les esters méthyliques/éthyliques) pour conduire aux acides de formule générale **(II)** dans laquelle X représente le groupe -O-Y-CO-.

### Lorsque X = -CO-:

Dans ce cas, des voies de synthèse comme celles représentées dans les schémas 3 ou 4 ci-après peuvent le cas échéant être utilisées.

### i) X représente -CO- et W représente -S-, -O- ou une liaison :

Lorsque X représente -CO- et W représente -S-, -O- ou une liaison, schéma 3, les acides dérivés de la phénothiazine ou du carbazole de formule générale **(II)** peuvent être obtenus à partir des 2-acétylphénothiazines (p.ex. Pharmazie (1984), 39(1), 22-3 ; Bull. Soc. Chim. (1968), (7), 2832-42, Pharmazie (1966), 21(11), 645-9) ou des 2-acétylcarbazoles (p.ex. Heterocycles (1994), 39(2), 833-45 ; J. Indian Chem. Soc. (1985), 62(7), 534-6; J. Chem. Soc. Chem. Comm. (1985), (2), 86-7) de formule générale **(II.4)** qui sont N-acétylés à l'aide du chlorure d'acétyle, par chauffage à reflux dans du toluène, pour conduire aux intermédiaires **(II.5)** *(*J. Med. Chem. (1998), 41(2), 148-156). Les intermédiaires **(II.5)** ainsi obtenus sont successivement traités par un mélange d'iode et de pyridine *(*J. Amer. Chem. Soc. (1944), 66, 894-895) et par de la soude aqueuse, à 100 °C, pour conduire aux acides carboxyliques de formule générale **(II)**.

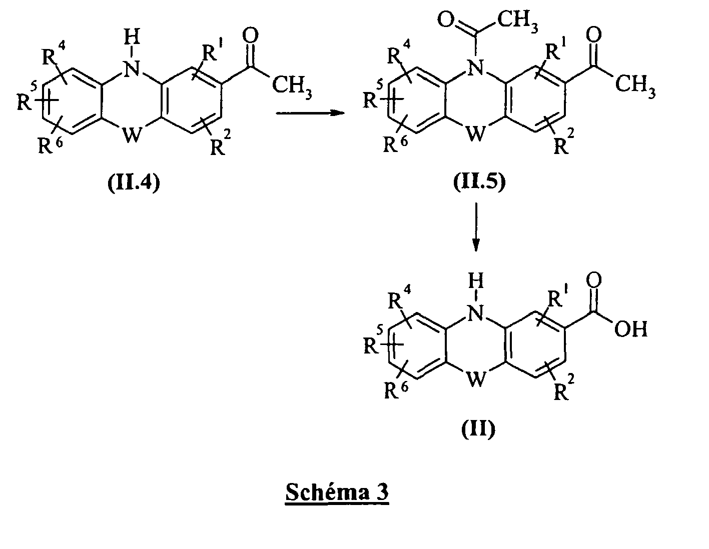

### ii) X représente -CO- et W représente -CH₂-CH₂- ou -CH=CH- :

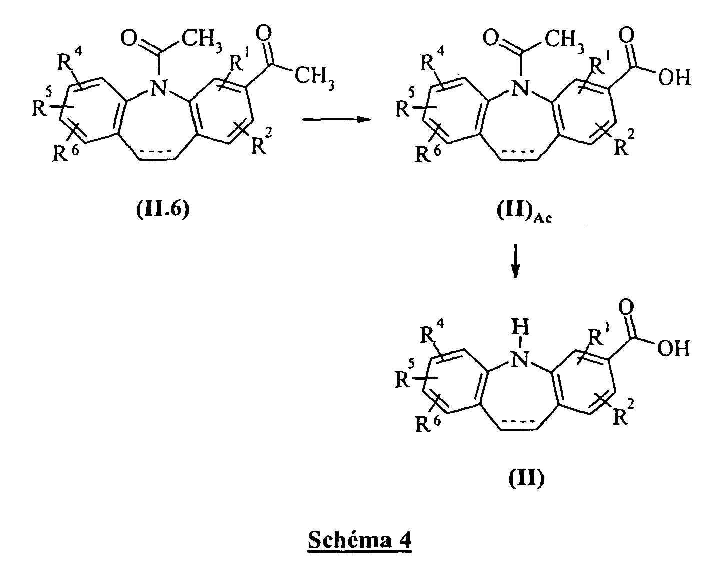

Lorsque X représente -CO- et W représente -CH₂-CH₂- ou -CH=CH-, schéma 4, les acides de formule générale **(II)_{Ac}** ou **(II)** peuvent être préparés à partir des dérivés di-acétylés de formule générale **(II.6)** (p.ex. J. Chem. Soc. (1973), 859-863). Comme dans le cas des phénothiazines (schéma 3), l'oxydation de l'acétyle est effectuée à l'aide d'iode et de pyridine poursuivie par une hydrolyse dans la soude aqueuse, à chaud. Les composé de formule générale **(II)_{Ac}** ainsi obtenus (qui sont des composés de formule générale **(II)** dans lesquels R³ représente un groupe acétyle) peuvent éventuellement subir un traitement supplémentaire en présence de potasse aqueuse, à reflux, pendant un temps de préférence compris entre 15 et 36 heures pour conduire aux acides carboxyliques de formule générale **(II).**

### Lorsque X=- Y-CO- :

Pour X = -Y-CO-, deux voies synthétiques représentées dans le schéma 5 ci-après permettent d'accéder aux acides carboxyliques de formule générale **(II),** selon la disponibilité des réactifs de départ.

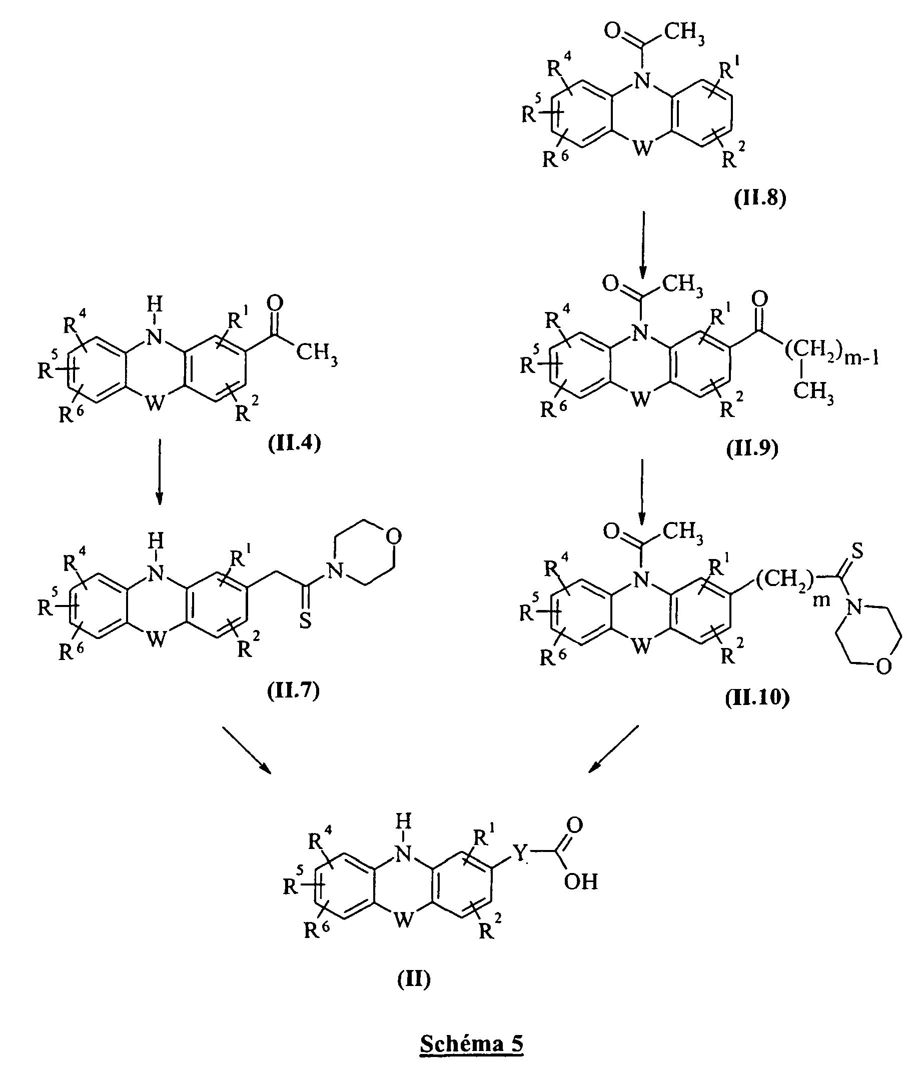

Dans le cas des 2-acétylphénothiazines (W = -S-) ou 2-acétylcarbazoles (W représente une liaison) de formule générale **(II.4),** précédemment décrits, la conversion en acide carboxylique de formule générale **(II)** est effectuée (partie gauche du schéma 5) par une réaction d'homologation de type Willgerodt-Kindler *(*Synthesis (1975), 358-375). Le chauffage des intermédiaires de formule générale **(II.4)** en présence de soufre et de morpholine conduit à la formation des thiocarboxamides de formule générale **(II.7)** (demandes de brevet allemand DE 2702714 et DE 1910291) qui sont convertis par hydrolyse en acides carboxyliques de formule générale **(II)**.

Alternativement (partie droite du schéma 5), lorsque W représente -S- ou une liaison et m est un entier supérieur à 1, ou bien lorsque W représente -CH₂-CH₂- ou -CH=CH- et m est un entier supérieur ou égal à 1, la synthèse des acides carboxyliques de formule générale **(II)** débute par l'acylation du noyau aromatique des intermédiaires de formule générale **(II.8)** par un chlorure d'alcanoyle dans CS₂, selon les conditions de la réaction de Friedel-Crafts *(*J. Amer. Chem. Soc. (1946), 68, 2673-78 ; J. Chem. Soc. Perkin Trans. 1 (1973), 859-861). Les intermédiaires acylés de formule générale **(II.9)** sont ensuite convertis en dérivés thiocarboxamides de formule générale **(II.10),** par la réaction de Willgerodt-Kindler, et finalement en acides carboxyliques de formule générale **(II)** selon la séquence chimique précédemment décrite.

### Préparation des intermédiaires de formule générale (III) :

Les dérivés amino-lactols de formule générale **(III)**, dans lesquels AA, R et n sont tels que décrits ci-dessus, sont accessibles en utilisant la voie de préparation représentée dans le schéma 6 ci-après. Cette méthode permet de préparer les composés de formule générale **(III)** dans lesquels n = 2 (ci-après les composés de formule générale **(III₂)** et les composés de formule générale **(III)** dans lesquels n = 3 (ci-après les composés de formule générale **(III)₃).**

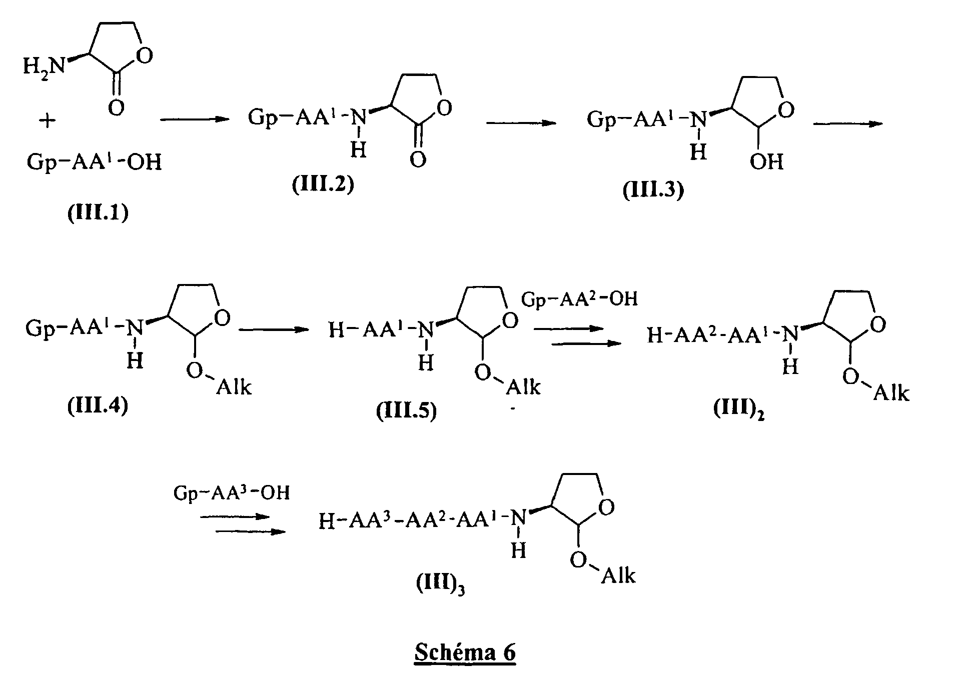

Les dérivés d'amino-butyrolactone de formule générale **(III.2)** sont obtenus par condensation des aminoacides protégés de formule générale **(III.1),** dans laquelle AA¹ est un radical d'acide aminé AA tel que précédemment défini dans la formule générale **(1)** et Gp est un groupe protecteur tel que, par exemple, un carbamate de benzyle ou de *tert*-butyle, sur la (S)-α-aminobutyrolactone dans les conditions classiques de la synthèse peptidique pour conduire aux carboxamides intermédiaires de formule générale **(III.2).** La lactone est ensuite réduite en lactol à l'aide d'un agent réducteur tel que, par exemple, l'hydrure de diisobutylaluminium (DIBAL), dans un solvant inerte tel que, par exemple, THF ou CH₂Cl₂, à une température de préférence inférieure à -50 °C, par exemple à environ -78° C. La fonction hémiacétalique des dérivés lactols de formule générale **(111.3)** est ensuite protégée en milieu alcoolique, par exemple dans du méthanol, à l'aide d'un acide fort tel que, par exemple, l'acide trifluoroacétique, pour conduire aux acétals de formule générale **(111.4).** La fonction amine des dérivés d'amino-acétals de formule générale **(III.4)** est ensuite déprotégée selon des méthodes décrites dans la littérature (T.W. Greene et P.G.M. Wuts, Protective Groups in Organic Synthesis, Second edition (Wiley-Interscience, 1991)). Les intermédiaires de formule générale **(III.5)** sont ensuite soumis à un ou deux cycles d'élongation-déprotection successifs de la chaîne peptidique, comme précédemment décrit, pour obtenir les dérivés di- (n = 2) ou tri-peptidiques (n = 3) de formules générales respectives **(III)₂** et **(III)₃.**

Dans le cas particulier où le groupe (AA²-AA¹) ou le groupe (AA³-AA²) est remplacé par un carbapeptide de formule générale NR¹⁷-(CH₂)₃-CH(R¹⁸)-CO-, les dérivés de formule générale **(III)₂** et **(III)₃** peuvent être obtenus selon une stratégie de synthèse peptidique analogue à celle décrite précédemment représentée dans le schéma 7 ci-après (dans lequel est uniquement représentée la situation où le groupe (AA²-AA¹) est remplacé par un carbapeptide - une méthode de préparation analogue pourra être utilisée pour le cas où le groupe (AA³-AA²) est remplacé par un carbapeptide).

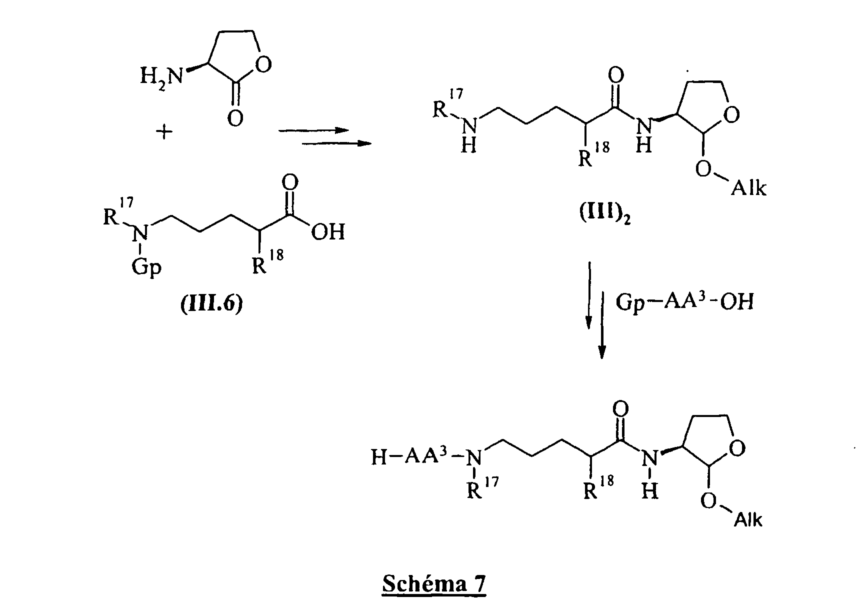

Les carbapeptides de formule générale **(III.6)** sont, quant à eux, accessibles à partir de méthodes décrites dans la littérature (p. ex. Int. J. Peptide Protein Res. (1992), 39, 273-277).

A moins qu'ils ne soient définis d'une autre manière, tous les termes techniques et scientifiques utilisés ici ont la même signification que celle couramment comprise par un spécialiste ordinaire du domaine auquel appartient cette invention.

### EXEMPLES

### Exemple 1 : N-(10H-phénothiazin-2-ylcarbonyl)-L-leucyl-L-leucyl-N¹-[(3S)-2-méthoxytétrahydrofuran-3-yl]-L-leucinamide :

### 1.1) N²-[(benzyloxy)carbonyl]-N¹-(3S)-2-oxotétrahydrofuran-3 yl]-L-leucinamide :

On dissout, dans 60 ml de DMF anhydre, 3,51 g (13,25 mmol) de Cbz-L-Leucine, 2,41 g (1 éq.) de bromhydrate de (S)-2-amino-4-butyrolactone, 1,97 g de HOBT (1,1 éq.) et 5,59 g (2,2 éq.) de chlorhydrate de 1-(3-diméthylaminopropyl)-3-éthylcarbodiimide (EDC) puis on ajoute 7,64 ml (3,3 éq.) de *N*,*N*-diisopropyléthylamine. Le mélange réactionnel est agité pendant 15 heures à 20 °C avant d'être versé dans 200 ml d'un mélange 1/1 d'acétate d'éthyle/eau. Après agitation et décantation, la solution organique est lavée successivement avec 100 ml d'une solution saturée de NaHCO₃, 50 ml d'eau, 100 ml d'une solution 1*M* d'acide citrique et finalement 100 ml d'une solution de saumure. La phase organique est séchée sur sulfate de sodium, filtrée et concentrée à sec sous vide. L'huile obtenue est lavée à l'aide d'isopentane et cristallisée ensuite dans un mélange dichlorométhane/isopentane. On obtient un solide blanc avec un rendement de 68%. Point de fusion : 130-131 °C.

### 1.2) N²-[(benzyloxy)carbonyl]-N¹-[(3S)-2-hydroxytétrahydrofuran-3-yl]-L-leucinamide :

Sous argon, dans un tricol contenant 60 ml de dichlorométhane anhydre, on dissout 1,24 g (3,56 mmol) de l'intermédiaire 1.1. L'ensemble est refroidi à -60 °C avant l'addition, goutte-à-goutte, de 10,7 ml (3 éq.) d'une solution 1*M* de DIBAL dans le dichlorométhane. A la fin de l'addition, le bain réfrigérant est enlevé et l'agitation est maintenue pendant 15 minutes supplémentaires. Le milieu réactionnel est alors versé, avec précaution, dans 100 ml d'une solution de sel de Rochelle à 20%. Après 2 heures d'agitation vigoureuse, 100 ml de dichlorométhane sont ajoutés et le tout est versé dans une ampoule à décanter. La phase organique est récupérée et lavée avec 50 ml d'eau et 50 ml de saumure. Après séchage sur sulfate de sodium et filtration, la solution organique est concentrée à sec sous vide. Le résidu d'évaporation est purifié sur une colonne de silice (éluant : acétate d'éthyle/heptane en proportions de 1/1 jusqu'à 8/2). On obtient un solide blanc avec un rendement de 72%. Point de fusion : 48-49 °C.

### 1.3) N²-[(benzyloxy)carbonyl]-N¹-[(3S)-2-méthoxytétrahydrofuran-3-yl]-L-leucinamide :

On ajoute, goutte-à-goutte à 20 °C, un excès d'acide trifluoroacétique (5 ml) à une solution de 0,82 g (2,34 mmol) de l'intermédiaire 1.2 dans 50 ml de méthanol. L'agitation est maintenue 15 heures à 20 °C. Le mélange réactionnel est ensuite partiellement concentré sous vide et repris dans 50 ml de dichlorométhane. La solution organique est lavée successivement avec 50 ml d'une solution saturée de NaHCO₃, 50 ml d'eau et 50 ml de saumure. Après séchage sur sulfate de sodium, filtration et concentration sous vide, le résidu d'évaporation est purifié sur une colonne de silice (éluant : acétate d'éthyle/heptane en proportions de 1/1 jusqu'à 7/3). On obtient un solide blanc avec un rendement de 80%. Point de fusion : 112-113°C.

### 1.4) N¹-[(3S)-2-méthoxytétrahydrofuran-3-yl]-L-leucinamide:

Dans un réacteur en inox contenant 60 ml de méthanol, on introduit 2 g (5,5 mmol) de l'intermédiaire 1.3 et 600 mg de Pd/C à 10%. Le mélange est agité sous 2 atm. de pression d'hydrogène pendant 1 heure. Après filtration du catalyseur, le méthanol est évaporé sous vide. Le résidu huileux obtenu (1,20 g ; 94%) est utilisé tel quel dans l'étape suivante.

### 1.5) N-[(benzyloxy)carbonyl]-L-leucyl-N¹-[(3S)-2-méthoxytétrahydrofuran-3-yl]-L-leucinamide :

Le protocole expérimental utilisé est le même que celui décrit pour la synthèse de l'intermédiaire 1.1, l'intermédiaire 1.4 remplaçant le bromhydrate de (S)-2-amino-4-butyrolactone. Le produit de la réaction est purifié sur une colonne de silice (éluant : acétate d'éthyle/heptane 7/3). On obtient 1,04 g d'un solide blanc avec un rendement de 69%. Point de fusion : 76-77 °C.

### 1.6) L-leucyl-N¹-[(3S)-2-méthoxytétrahydrofuran-3-yl]-L-leucinamide :

Le protocole expérimental utilisé est le même que celui décrit pour la synthèse de l'intermédiaire 1.4, l'intermédiaire 1.5 remplaçant l'intermédiaire 1.3. Le produit de la réaction est utilisé sans purification supplémentaire. On obtient 0,74 g d'une mousse incolore avec un rendement de 96%.

### 1.7) N-[(benzyloxy)carbonyl]-L-leucyl-L-leucyl-N¹-[(3S)-2-méthoxytétrahydrofuran-3 yl]-L-leucinamide :

Le protocole expérimental utilisé est le même que celui décrit pour la synthèse de l'intermédiaire 1.1, l'intermédiaire 1.6 remplaçant le bromhydrate de (S)-2-amino-4-butyrolactone. Le produit de la réaction est cristallisé dans un mélange acétate d'éthyle/isopentane. On obtient 0,96 g d'un solide blanc avec un rendement de 77%. Point de fusion : 210-212 °C.

### 1.8) L-leucyl-L-leucyl-N¹-[(3S)-2-méthoxytétrahydrofueran-3 yl]-L-leucinamide :

Le protocole expérimental utilisé est le même que celui décrit pour la synthèse de l'intermédiaire 1.4, l'intermédiaire 1.7 remplaçant l'intermédiaire 1.3. On obtient 0,74 g d'un solide blanc avec un rendement quantitatif. Point de fusion : 187-188 °C.

### 1.9) 1-(10-acétyl-10H-phénothiazin-2-yl)éthanone :

Dans un ballon de 500 ml contenant 150 ml de toluène, on introduit 30 g (0,118 mol) de 2-acétylphénothiazine et 9,28 g (1 éq.) de chlorure d'acétyle. Le mélange réactionnel est chauffé à reflux pendant 45 minutes avant l'introduction d'une nouvelle portion de 4,6 g (0,5 éq.) de chlorure d'acétyle. L'agitation est maintenue sous chauffage à reflux pendant 2 heures supplémentaires. Le milieu réactionnel est ensuite versé sur environ 200 g de glace. Après agitation, la phase organique est décantée et lavée successivement avec 100 ml d'eau et 100 ml de saumure. La solution organique est séchée sur sulfate de sodium, filtrée et concentrée à sec à l'aide d'un évaporateur rotatif. On obtient un solide jaune (33 g; 100%) qui est utilisé dans l'étape suivante sans purification supplémentaire.

### 1.10) acide 10H-phénothiazine-2-carboxylique:

On dissout 24 g (0,084 mol) de l'intermédiaire 1.9 dans 55 ml de pyridine. Après addition de 20,32 g (1 éq.) d'iode, le mélange réactionnel est chauffé à 100 °C pendant 15 minutes. L'agitation est ensuite maintenue 20 heures supplémentaires à 20 °C avant concentration à sec à l'aide d'un évaporateur rotatif. Au résidu d'évaporation ainsi obtenu, on ajoute 100 ml d'eau suivi de 15 g (4,46 éq.) de NaOH en pastilles. Ce mélange est ensuite chauffé à 100 °C pendant 1 heure. Après refroidissement à l'aide d'un bain de glace, le mélange réactionnel est lavé par 100 ml d'acétate d'éthyle. La solution aqueuse est ensuite acidifiée à l'aide de 50 ml d'une solution aqueuse 12*N* de HCl, un précipité abondant apparaît. Celui-ci est filtré sur Büchner, lavé à l'aide d'éthanol et séché sous vide à 75 °C.
Une portion supplémentaire du produit attendu peut être récupérée à partir du filtrat acide. Celui-ci est extrait à l'aide de 2 fois 100 ml d'acétate d'éthyle. La phase organique est ensuite lavée avec 50 ml d'eau suivi de 50 ml de saumure. Après séchage sur sulfate de sodium, la solution organique est concentrée à sec sous vide. La quantité totale recueillie est de 16,8 g sous la forme d'un solide jaune avec un rendement global de 82%. Point de fusion : > 235 °C.

### 1.11) N-(10H-phénothiazin-2-ylcarbonyl)-L-leucyl-L-leucyl-N¹-[(3S)-2-méthoxytétrahydrofuran -3-yl]-L-leucinamide :

Le protocole expérimental de cette condensation peptidique est le même que celui décrit pour la synthèse de l'intermédiaire 1.1, en utilisant cette fois l'acide 10*H* phénothiazine-2-carboxylique (intermédiaire 1.10) et le L-leucyl-L-leucyl-N¹-[(3S)-2-méthoxytétrahydrofuran-3-yl]-L-leucinamide (intermédiaire 1.8). Le produit de la réaction est purifié par chromatographie sur silice (éluant : acétate d'éthyle/heptane en proportions de 7/3 jusqu'à 1/1). On obtient 228 mg d'un solide jaune avec un rendement de 21%. Point de fusion : 164-165 °C.

### Exemple 2 : N-(10H-phénothiazin-2-ylcarbonyl)-L-leucyl-L-leucyl-N¹-[(3S)-2-hydroxytétrahydrofuran-3-yl]-L-leucinamide :

Dans un ballon contenant 12 ml d'acétone, on dissout 228 mg (0,33 mmol) du composé de l'exemple 1. On ajoute ensuite, à 20 °C, goutte-à-goutte, 2 ml d'une solution aqueuse de HCl 2*N*. L'agitation est maintenue pendant 6 h 30. Le milieu réactionnel est ensuite concentré à sec et le résidu d'évaporation est purifié par chromatographie sur une colonne de silice (éluant : acétate d'éthyle/heptane 9/1). On obtient 49 mg d'un solide jaune avec un rendement de 22%. Point de fusion : 174-176 °C.

*Les composés des exemples 3 à 6 ont été préparés selon la même stratégie de synthèse que celle décrite pour le composé de l'exemple 1 à partir des intermédiaires 1.4, 1.6 et 1.10 et des aminoacides protégés commerciaux appropriés.*

### Exemple 3 : N-(10H-phénothiazin-2-ylcarbonyl)glycyl-N¹-((3S)-2-méthoxytétrahydrofuran-3-yl]-L-leucinamide :

*Solide jaune.*

### Exemple 4 : N-(10H-phénothiazin-2-ylcarbonyl)leucyl-N¹-[(3S)-2-méthoxytétrahydrofuran-3-yl]-L-leucinamide :

Solide jaune. Point de fusion : 180-180,5 °C.

### Exemple 5 : N⁶-[(benzyloxy)carbonyl]-N²-(10H-phénothiazin-2-ylcarbonyl)lysyl-N¹-[(3S)-2-méthoxytétrahydrofuran-3-yl]-L-leucinamide :

Solide jaune. Point de fusion : 102-103 °C.

### Exemple 6 : 1-(10H-phénothiazin-2-ylcarbonyl)-L-prolyl-N¹-[(3S)-2-méthoxytétrahydrofuran-3-yl]-L-leucinamide :

Solide jaune. Point de fusion : 243-243,5 °C.

*Les composés des exemples 7, 8, 9 et 10 ont été préparés selon le protocole expérimental décrit pour le composé de l'exemple 2 à partir des composés des exemples 3, 4,* 5 *et 6 respectivement.*

### Exemple 7 : N-(10H-phénothiazin-2-ylcarbonyl)glycyl-N¹-[(3S)-2-hydroxytétrahydrofuran-3-yl]-L-leucinamide :

Solide jaune. Point de fusion : 126-126,5 °C.

### Exemple 8 : N-(10H-phénothiazin-2-ylcarbonyl)leucyl-N¹-[(3S)-2-hydroxytétrahydrofuran-3-yl]-L-leucinamide :

Solide jaune-vert. Point de fusion : 144-144,5 °C.

### Exemple 9 : N⁶-[(benzyloxy)carbonyl]-N²-(10H-phénothiazin-2-ylcarbonyl)lysyl-N¹-[(3S)-2-hydroxytétrahydrofuran-3-yl]-L-leucinamide :

Solide jaune. Point de fusion : 109-109,5 °C.

### Exemple 10 : 1-(10H-phénothiazin-2-ylcarbonyl)-L-prolyl-N¹-[(3S)-2-hydroxytétrahydrofuran-3-yl]-L-leucinamide :

Solide jaune pâle. Point de fusion : 144,5-145 °C.

### Exemple 11 : N-(10H-phénothiazin-2-ylcarbonyl)leucyl-N¹-[(3S)-2-(acétyloxy)-tétrahydrofuran-3-yl]-L-leucinamide :

A une solution de 366 mg (0,66 mmol) du composé de l'exemple 8 dans 6 ml de dichlorométhane, on ajoute 0,62 ml (10 éq.) d'anhydride acétique et 40 mg (0,5 éq.) de 4-diméthylaminopyridine. Le mélange est agité pendant 4 heures à 20 °C. La solution est ensuite diluée par 20 ml de dichlorométhane et 20 ml d'une solution saturée de NaHCO₃. Après agitation et décantation, la phase organique est lavée par 20 ml de saumure. La solution organique est ensuite séchée sur sulfate de sodium, filtrée et concentrée à sec sous vide. Le résidu d'évaporation est purifié sur une colonne de silice (éluant : heptane/AcOEt : 4/6 jusqu'à 0/1). Les fractions pures sont collectées et concentrées sous vide. Le produit est cristallisé dans un mélange dichlorométhane/isopentane. Les cristaux sont filtrés, rincés à l'isopentane et séchés. On obtient 180 mg d'un solide jaune pâle avec un rendement de 56%. Point de fusion : 121-122 °C.

### Exemple 12 : trifluoroacétate de N²-(10H-phénothiazin-2-ylcarbonyl)lysyl-N¹-[(3S)-2-hydroxytétrahydrofuran-3-yl]-L-leucinamide :

On dissout 0,5 g (0,7 mmole) du composé de l'exemple 9 dans 5 ml d'acide acétique. Après refroidissement à l'aide d'un bain de glace, on ajoute goutte-à-goutte 5 ml d'une solution aqueuse à 33% de HBr. Après 4 heures d'agitation à 20 °C, le mélange réactionnel est concentré à sec et le résidu d'évaporation est purifié par HPLC préparative au moyen d'une colonne C18, 5 µm (éluant : THF/H₂O/TFA : 40/60/0,02). Après lyophilisation, on obtient 100 mg d'un solide beige avec un rendement de 2 1 %.

### Exemple 13 : N-(10H-phénothiazin-2-ylacétyl)-L-leucyl-N¹-[(3S)-2-méthoxytétrahydrofuran-3-yl]-L-leucinamide :

### 13.1) 2-(2-morpholin-4-yl-2-thioxoéthyl)-10H phénothiazine :

Le protocole expérimental pour la préparation de cet intermédiaire est inspiré d'une synthèse décrite dans la demande de brevet allemand DE 2 702 714. Dans un tricol équipé d'un thermomètre, d'un réfrigérant et d'une sortie de gaz plongeant successivement dans un piège de soude 2*N* et un piège d'une solution concentrée de permanganate de potassium, on introduit 24,13 g (0,1 mol) de 2-acétylphénothiazine, 5,13 g (1,6 éq.) de soufre et 39 ml (4,5 éq.) de morpholine. Le mélange réactionnel est chauffé à reflux (température interne = 119°C) pendant 15 heures. Après refroidissement, cette solution brune est versée, sous agitation, dans 300 ml d'éthanol absolu. Le mélange est stocké 1 heure à 4 °C pour initier la cristallisation et ensuite une nuit à -18 °C. Le solide obtenu est alors filtré et rincé à l'aide d'éthanol froid et d'heptane. Après séchage, on obtient 27 g d'un solide orange avec un rendement de 79%.

### 13.2) acide 10H-phénothiazin-2-ylacétique :

Dans un tricol équipé comme précédemment décrit, on introduit 31,23 g (91,2 mmol) de l'intermédiaire 13.1, 36 g (6 éq.) de potasse à 85% et 350 ml d'éthanol absolu. Le mélange réactionnel est chauffé à reflux pendant 15 heures. Après refroidissement, ce mélange est concentré de moitié sous vide et ensuite versé dans 650 ml d'eau. Sous agitation, on ajoute de l'acide sulfurique concentré jusqu'à pH 1 et ensuite l'ensemble est porté à 80 °C. Après 2 heures de chauffage, le mélange est refroidi, le précipité est filtré et rincé par 4 fois 40 ml d'eau. Le solide ainsi obtenu est dissous dans de l'acétone et filtré pour éliminer un insoluble. Le filtrat est alors concentré à sec sous vide pour conduire à une poudre jaune pâle (15,67 g) avec un rendement de 67%. Point de fusion : 201,5-202 °C.

### 13.3) N-(10H-phénothiazin-2-ylacétyl)-L-leucyl-N¹-[(3S)-2-méthoxytétrahydrofuran-3-yl]-L-leucinamide:

Le protocole expérimental utilisé est le même que celui décrit pour l'étape 1.11 de l'exemple 1, à partir des intermédiaires 1.6 et 13.2. Solide beige. Point de fusion: 216-216,5 °C.

### Exemple 14 : O-(tert-butyl)-N-(10H-phénothiazin-2-ylacétyl)-L-séryl-N¹-[(3S)-2-méthoxytétrahydrofuran-3-yl]-L-leucinamide :

### 14.1) O-(tert-butyl)-L-séryl-N¹-[(3S)-2-méthoxytétrahydrofuran-3 yl]-L-leucinamide :

Cet intermédiaire a été préparé en 2 étapes selon les protocoles expérimentaux décrits pour la synthèse des intermédiaires 1.5 et 1.6, à partir de l'intermédiaire 1.4 et de la Cbz-L-sérine(tBu) commerciale. On obtient une huile incolore.

### 14.2) O-(tert-butyl)-N-(10H phénothiazin-2 ylacétyl)-L-séryl-N¹-[(3S)-2-méthoxytétrahydrofuran-3 yl]-L-leucinamide :

Le protocole expérimental utilisé est le même que celui décrit pour l'étape 1.11 de l'exemple 1, à partir des intermédiaires 14.1 et 13.2. Solide blanc. Point de fusion: 179-180°C.

### Exemple 15 : N-(10H-phénothiazin-2-ylacétyl)-L-alanyl-3-cyclohexyl-N¹-[(3S)-2-méthoxytétrahydrofuran-3-yl]-L-alaninamide :

### 15.1) 3-cyclohexyl-N¹-[(3S)-2-méthoxytétrahydrofuran-3 yl]-L-alaninamide :

Cet intermédiaire a été préparé en 4 étapes selon les protocoles expérimentaux décrits pour les étapes 1.1 à 1.4 de l'exemple 1, à partir du bromhydrate de (*S*)-2-amino-4-butyrolactone et de la Cbz-3-cyclohexyl-L-alanine, commerciaux. On obtient une huile incolore.

### 15.2) L-alanyl-3-cyclohexyl-N¹-[(3S)-2-mélhoxytétrahydrofuran-3 yl]-L-alaninamide :

Cet intermédiaire a été préparé en 2 étapes selon les protocoles expérimentaux décrits pour la synthèse des intermédiaires 1.5 et 1.6, à partir de l'intermédiaire 15.1 et de la Cbz-L-alanine.

### 15.3) N-(10H-phénothiazin-2-ylacétyl)-L-alanyl-3-cyclohexyl-N¹-[(3S)-2-méthoxytétrahydrofuran-3-yl]-L-alaninamide :

Le protocole expérimental utilisé est le même que celui décrit pour l'étape 1.11 de l'exemple 1, à partir des intermédiaires 15.2 et 13.2. On obtient un solide beige. Point de fusion : 225-226 °C.

*Les composés des exemples 16, 17 et 18 ont été préparés selon le protocole expérimental décrit pour le composé de l'exemple 2 à partir des composés des exemples 13, 14 et 15 respectivement.*

### Exemple 16 : N-(10H-phénothiazin-2-ylacétyl)-L-leucyl-N¹-[(3S)-2-hydroxytétrahydrofuran-3-yl]-L-leucinamide :

Solide beige. Point de fusion : 168-168,5 °C.

### Exemple 17 : O-(tert-butyl)-N-(10H-phénothiazin-2-ylacétyl)-L-séryl-N¹-[(3S)-2-hydroxytétrahydrofuran-3-yl]-L-leucinamide :

Solide beige clair. Point de fusion : 135-136 °C.

### Exemple 18 : N-(10H-phénothiazin-2-ylacétyl)-L-alanyl-3-cyclohexyl-N¹-[(3S)-2-hydroxytétrahydrofuran-3-yl]-L-alaninamide :

Solide blanc. Point de fusion : 215-217 °C.

### Exemple 19 : N-[3-(10H-phénothiazin-2-yl)propanoyl]-L-leucyl-N¹-[(3S)-2-méthoxytétrahydrofuran-3-yl]-L-leucinamide :

### 19.1) 10-acétyl-10H phénothiazine :

Dans un ballon de 500 ml contenant 200 ml de toluène, on ajoute 20 g (0,1 mol) de phénothiazine suivi de 14,3 ml (2 éq.) de chlorure d'acétyle. Le mélange réactionnel hétérogène est agité pendant 1 heure à 50 °C. Après concentration à sec, le précipité est repris dans un minimum d'isopentane et filtré. Après séchage on obtient 24 g d'un solide beige avec un rendement quantitatif. Point de fusion : 210-211 °C.

### 19.2) 7-(10-acétyl-10H-phénothiazin-2-yl)propan-1-one :

Dans un ballon multicol équipé d'un agitateur mécanique, d'une arrivée d'argon, d'un réfrigérant et d'une ampoule à addition, on introduit 150 ml de CS₂ suivi de 24 g de l'intermédiaire 19.1. A cette suspension, agitée vigoureusement, on ajoute, goutte-à-goutte, 10,4 ml (1,2 éq.) de chlorure de propionyle suivi de 50 g (4 éq.) de AlCl₃ en une portion, à l'aide d'un entonnoir à solide. L'entonnoir est immédiatement rincé avec 50 ml supplémentaires de CS₂. Tout en agitant vigoureusement, le mélange est chauffé à 55 °C pendant 2 heures. L'ensemble est ensuite refroidi avec un bain de glace et le CS₂ est éliminé à l'aide d'une canule. L'hydrolyse du mélange réactionnel débute par l'addition progressive de petits morceaux de glace et quand la réaction est moins virulente l'hydrolyse est achevée à l'aide d'eau froide. L'ensemble est finalement dilué à l'aide de 300 ml d'acétate d'éthyle et transféré dans une ampoule à décanter. La solution organique est décantée, lavée par 2 fois 100 ml d'eau et 100 ml de saumure. Après séchage sur sulfate de sodium, filtration et concentration à sec sous vide, le résidu obtenu est trituré dans de l'isopentane. Ce solide est alors filtré et rincé par un minimum d'isopentane. On obtient 13,2 g d'un solide blanc avec un rendement de 45%. Point de fusion : 146,5-147 °C.

### 19.3) acide 3-(10H-phénothiazin-2-yl)propanoique :

Les protocoles expérimentaux utilisés sont les mêmes que ceux décrits pour les synthèses des intermédiaires 13.1 et 13.2. A partir de 6 g de l'intermédiaire 19.2, on obtient 2 g d'un solide brun avec un rendement global (2 étapes) de 33%. Ce composé (pureté de 80%) est utilisé tel quel dans l'étape suivante.

### 19.4) N-[3-(10H-phénothiazin-2-yl)propanoyl]-L-leucyl. N¹-[(3S) -2-méthoxytétrahydrofuran -3-yl]-L-leucinamide :

Cet exemple a été préparé selon la même stratégie de synthèse que celle décrite pour l'étape 1.11 de l'exemple 1 à partir des intermédiaires 1.6 et 19.3. Solide jaune. Point de fusion : 215-216 °C.

### Exemple 20 : N-[3-(10H-phénothiazin-2-yl)propanoyl]-L-leucyl-N¹-[(3S)-2-hydroxytétrahydrofuran-3-yl]-L-leucinamide :

Le protocole expérimental utilisé est le même que celui décrit pour le composé de l'exemple 2, en partant du composé de l'exemple 19. Solide beige clair. Point de fusion : 212-213 °C.

### Exemple 21 : N-[(10H-phénothiazin-2-yloxy)acétyl]-L-leucyl-N¹-[(3S)-2-méthoxytétrahydrofuran-3-yl]-L-leucinamide :

### 21.1) 10H phénothiazin-2-ol :

On chauffe à 170 °C pendant 15 heures un mélange de 25 g (109 mmol) de 2-méthoxyphénothiazine et de 60 g (4,8 éq.) de chlorure de pyridinium. Après refroidissement à une température d'environ 80 °C, la solution brune obtenue est diluée avec 200 ml d'acétate d'éthyle. L'agitation est maintenue 30 minutes avant de verser le mélange réactionnel dans 200 ml d'eau. Après agitation et décantation, la phase organique est séchée sur sulfate de sodium et concentrée à sec, sous vide. Le solide verdâtre obtenu est recristallisé dans 700 ml de toluène bouillant. Un insoluble est éliminé par filtration, le filtrat cristallise spontanément pendant la nuit. Les cristaux sont collectés par filtration et rincés à l'aide d'isopentane. Après séchage, on obtient 12,43 g d'un solide gris avec un rendement de 53%. Point de fusion : 207-208 °C.

### 21.2) (10H-phénothiazin-2-yloxy)acétate de tert-butyle :

Dans un ballon de 100 ml, on dissout 1 g (4,6 mmol) de l'intermédiaire 21.1 et 1,40 ml (2 éq.) de bromoacétate de tertiobutyle dans 25 ml de THF. A cette solution, on ajoute 1,93 g (3 éq.) de K₂CO₃ et le mélange réactionnel est chauffé à reflux pendant 15 heures. Après refroidissement, on ajoute 50 ml d'eau et 50 ml de dichlorométhane. La phase organique est décantée, lavée par 50 ml d'eau et 50 ml de saumure. Après séchage sur sulfate de sodium, filtration et concentration sous vide, le résidu obtenu est purifié sur une colonne de silice, éluant acétate d'éthyle/heptane (2/8). On obtient 940 mg d'un solide crème avec un rendement de 70%.

### 21.3) acide (10H-phénothiazin-2-yloxy)acétique :

On dissout 935 mg (2,8 mmol) de l'intermédiaire 21.2 dans 9 ml de dichlorométhane. Le mélange est refroidi à 0 °C avant l'addition, goutte-à-goutte, de 2,19 ml (10 éq.) d'acide trifluoroacétique. A la fin de l'addition, on laisse la température remonter à 20°C et l'agitation est maintenue pendant 3 heures. Le mélange réactionnel est ensuite concentré à sec sous vide et le résidu redilué par 50 ml d'acétate d'éthyle. La solution organique est lavée par 2 fois 25 ml d'eau suivi de 25 ml de saumure. Après séchage sur sulfate de sodium, filtration et concentration à l'évaporateur rotatif, on obtient 540 mg d'un solide rosé avec un rendement de 69%. Point de fusion : 180-181 °C.

### 21.4) N-[(10H-phénothiazin-2-yloxy)acétyl]-L-leucyl-N¹-((3S)-2-méthoxytétrahydrofuran-3 yl]-L-leucinamide :

Cet exemple a été préparé selon la même stratégie de synthèse que celle décrite pour l'étape 1.11 de l'exemple 1 à partir des intermédiaires 1.6 et 21.3. Solide beige. Point de fusion : 211,5-212 °C.

*Les composés des exemples 22 à 35 ont été préparés selon la même stratégie de synthèse que celle décrite pour le composé de l'exemple 1 à partir des intermédiaires 1.4, 21.3 et des aminoacides protégés commerciaux appropriés.*

### Exemple 22 : N-[(10H-phénothiazin-2-yloxy)acétyl]-glycyl-N¹-[(3S)-2-méthoxytétrahydrofuran-3-yl]-L-leucinamide :

Solide beige. Point de fusion : 181-182 °C.

### Exemple 23 : N-[(10H-phénothiazin-2-yloxy)acétyl]-L-alanyl-N¹-[(3S)-2-méthoxytétrahydrofuran-3-yl]-L-leucinamide :

Solide blanc. Point de fusion : 195-196 °C.

### Exemple 24 : N-[(10H-phénothiazin-2-yloxy)acétyl]-L-valyl-N¹-[(3S)-2-méthoxytétrahydrofuran-3-yl]-L-leucinamide :

Solide blanc. Point de fusion : 240-241 °C.

### Exemple 25 : N-[(10H-phénothiazin-2-yloxy)acétyl]-β-alanyl-N¹-[(3S)-2-méthoxytétrahydrofuran-3-yl]-L-leucinamide :

Solide brun. Point de fusion : 155-157 °C.

### Exemple 26 : N-méthyl-N-[(10H-phénothiazin-2-yloxy)acétyl]glycyl-N¹-[(3S)-2-méthoxytétrahydrofuran-3-yl]-L-leucinamide :

Solide rose pâle. Point de fusion : 92-95 °C.

### Exemple 27 : N-[(10H-phénothiazin-2-yloxy)acétyl]-D-valyl-N¹-[(3S)-2-méthoxytétrahydrofuran-3-yl]-L-leucinamide :

Solide beige clair. Point de fusion : 204-206 °C.

### Exemple 28 : 3-méthyl-N-[(10H-phénothiazin-2-yloxy)acétyl]-L-valyl-N¹-[(3S)-2-méthoxytétrahydrofuran-3-yl]-L-leucinamide :

Solide beige. Point de fusion : 152-153 °C.

### Exemple 29 : N¹-[(3S)-2-méthoxytétrahydrofuran-3-yl]-N²-((2S)-2-{[(10H-phénothiazin-2-yloxy)-acétyl]amino}butanoyl)-L-leucinamide :

Solide beige. Point de fusion : 164-165 °C.

### Exemple 30 : N-[(10H-phénothiazin-2-yloxy)acétyl]-L-norvalyl-N¹-[(3S)-2-méthoxytétrahydrofuran-3-yl]-L-leucinamide :

Solide beige. Point de fusion : 225-226 °C.

### Exemple 31 : N-[(10H-phénothiazin-2-yloxy)acétyl]-L-séryl-N¹-[(3S)-2-méthoxytétrahydrofuran-3-yl]-L-leucinamide :

Solide gris.

### Exemple 32 : N-[(10H-phénothiazin-2-yloxy)acétyl]-L-thréonyl-N¹-[(3S)-2-méthoxytétrahydrofuran-3-yl]-L-leucinamide :

Solide jaune pâle. Point de fusion : 92-93 °C.

### Exemple 33 : N¹-[(3S)-2-méthoxytétrahydrofuran-3-yl]-N²-((2S)-2-{[(10H-phénothiazin-2-yloxy)acétyl]amino}-2-phényléthanoyl)-L-leucinamide :

Solide jaune pâle. Point de fusion : 215-217 °C.

### Exemple 34 : N¹-[(3S)-2-méthoxytétrahydrofuran-3-yl]-N²-((2S)-2-{[(10H-phénothiazin-2-yloxy)acétyl]amino}but-3-enoyl)-L-leucinamide :

Solide rose pâle.

### Exemple 35 : 2-méthyl-N-[(10H-phénothiazin-2-yloxy)acétyl]alanyl-N¹-[(3S)-2-méthoxytétrahydrofuran-3-yl]-L-leucinamide :

Solide rose pâle. Point de fusion : 119-120 °C.

### Exemple 36 : N-[(10H-phénothiazin-2-yloxy)acétyl]-glycyl-N¹-[(3S)-2-méthoxytétrahydrofuran-3-yl]-L-valinamide :

### 36.1) N¹-[(3S)-2-méthoxytétrahydrofuran-3 yl]-L-valinamide :

Cet intermédiaire a été préparé en 4 étapes selon les protocoles expérimentaux décrits pour les étapes 1.1 à 1.4 de l'exemple 1, en utilisant le bromhydrate de (*S*)-2-amino-4-butyrolactone et la Cbz-L-valine. On obtient une huile incolore.

### 36.2) N-[(10H-phénothiazin-2-yloxy)acétyl]-glycyl-N¹-[(3S)-2-méthoxytétrahydrofuran-3-yl]-L-valinamide:

Ce composé a été préparé en 3 étapes selon les protocoles expérimentaux décrits pour les étapes 1.5, 1.6 et 1.11 de l'exemple 1 en utilisant la Cbz-Glycine et les intermédiaires 36.1 et 21.3. On obtient un solide beige.

### Exemple 37 : N-[(10H-phénothiazin-2-yloxy)acétyl]-glycyl-3-cyclohexyl-N¹-[(3S)-2-méthoxytétrahydrofuran-3-yl]-L-alaninamide :

La stratégie de synthèse utilisée est la même que celle décrite pour l'étape 36.2 de l'exemple 36 en utilisant la Cbz-Glycine et les intermédiaires 15.1 et 21.3. On obtient un solide rose pâle. Point de fusion : 179-180 °C.

### Exemple 38 : N-[(10H-phénothiazin-2-yloxy)acétyl]-glycyl-N-[(3S)-2-méthoxytétrahydrofuran-3-yl]-L-phénylalaninamide :

### 38.1) N-[(3S)-2-méthoxytétrahydrofuran-3 yl]-L-phénylalaninamide :

Cet intermédiaire a été préparé en 4 étapes selon les protocoles expérimentaux décrits pour les étapes 1.1 à 1.4 de l'exemple 1, en utilisant le bromhydrate de (*S*)-2-amino-4-butyrolactone et la Cbz-L-phénylalanine. On obtient une huile jaune.

### 38.2) N-[(10H-phénothiazin-2-yloxy)acétyl]-glycyl-N-[(3S)-2-méthoxytétrahydrofuran-3-yl]-L-phénylalaninamide:

La stratégie de synthèse utilisée est la même que celle décrite pour l'étape 36.2 de l'exemple 36 en utilisant la Cbz-Glycine et les intermédiaires 38.1 et 21.3. On obtient un solide beige. Point de fusion : 203-204 °C.

### Exemple 39 : N-[(10H-phénothiazin-2-yloxy)acétyl]glycyl-N²-isobutyl-N¹-[(3S)-2-méthoxytétrahydrofuran-3-yl]glycinamide :

### 39.1) N²-isobutyl-N¹-[(3S)-2-méthoxytétrahydrofuran-3-yl]glycinamide :

Cet intermédiaire a été préparé en 4 étapes selon les protocoles expérimentaux décrits pour les étapes 1.1 à 1.4 de l'exemple 1, en utilisant le bromhydrate de (*S*)-2-amino-4-butyrolactone et la Boc-N-isobutylglycine *(*J. Med. Chem. (2000), 43(15), 2805-2813). On obtient une huile incolore.

### 39.2) N-[(10H-phénothiazin-2-yloxy)acétyl]glycyl-N²-isobutyl-N¹-[(3S)-2-méthoxytétrahydrofuran-3-yl]glycinamide :

La stratégie de synthèse utilisée est la même que celle décrite pour l'étape 36.2 de l'exemple 36, en utilisant la Cbz-Glycine et les intermédiaires 39.1 et 21.3. On obtient un solide beige. Point de fusion : 162-164 °C.

*Les composés des exemples 40 à 58 ont été préparés selon le protocole expérimental décrit pour le composé de l'exemple 2 à partir des composés des exemples 21 à 39 respectivement.*

### Exemple 40 : N-[(10H-phénothiazin-2-yloxy)acétyl]-L-leucyl-N¹-[(3S)-2-hydroxytétrahydrofuran-3-yl]-L-leucinamide :

Solide beige clair. Point de fusion : 141,5-142 °C.

### Exemple 41 : N-[(10H-phénothiazin-2-yloxy)acétyl]-glycyl-N¹-[(3S)-2-hydroxytétrahydrofuran-3-yl]-L-leucinamide :

Solide rose pâle. Point de fusion : 184-186 °C.

### Exemple 42 : N-[(10H-phénothiazin-2-yloxy)acétyl]-L-alanyl-N¹-[(3S)-2-hydroxytétrahydrofuran-3-yl)-L-leucinamide :

Solide beige. Point de fusion : 144-146 °C.

### Exemple 43 : N-[(10H-phénothiazin-2-yloxy)acétyl]-L-valyl-N¹-[(3S)-2-hydroxytétrahydrofuran-3-yl]-L-leucinamide :

Solide blanc. Point de fusion : 205-206 °C.

### Exemple 44 : N-[(10H-phénothiazin-2-yloxy)acétyl]-β-alanyl-N¹-[(3S)-2-hydroxytétrahydrofuran-3-yl]-L-leucinamide :

Solide rose pâle. Point de fusion : 161-162 °C.

### Exemple 45 : N-méthyl-N-[(10H-phénothiazin-2-yloxy)acétyl]glycyl-N¹-[(3S)-2-hydroxytétrahydrofuran-3-yl]-L-leucinamide :

Solide beige clair. Point de fusion : 137-138 °C.

### Exemple 46 : N-[(10H-phénothiazin-2-yloxy)acétyl]-D-valyl-N¹-[(3S)-2-hydroxytétrahydrofuran-3-yl]-L-leucinamide :

Solide blanc. Point de fusion : 145-146 °C.

### Exemple 47 : 3-méthyl-N-[(10H-phénothiazin-2-yloxy)acétyl]-L-valyl-N¹-[(3S)-2-hydroxytétrahydrofuran-3-yl]-L-leucinamide :

Solide blanc. Point de fusion : 157-159 °C.

### Exemple 48 : N¹-[(3S)-2-hydroxytétrahydrofuran-3-yl]-N²-((2S)-2-{[(10H-phénothiazin-2-yloxy)acétyl]amino}butanoyl)-L-leucinamide :

Solide beige. Point de fusion : 160-161 °C.

### Exemple 49 : N-[(10H-phénothiazin-2-yloxy)acétyl]-L-norvalyl-N¹-[(3S)-2-hydroxytétrahydrofuran-3-yl]-L-leucinamide :

Solide beige. Point de fusion : 195-196 °C.

### Exemple 50 : N-[(10H-phénothiazin-2-yloxy)acétyl]-L-séryl-N¹-[(3S)-2-hydroxytétrahydrofuran-3-yl]-L-leucinamide :

Solide gris. Point de fusion : 128-130 °C.

### Exemple 51 : N-[(10H-phénothiazin-2-yloxy)acétyl]-L-thréonyl-N¹-[(3S)-2-hydroxytétrahydrofuran-3-yl]-L-leucinamide :

Solide blanc. Point de fusion : 195-196 °C.

### Exemple 52 : N¹-[(3S)-2-hydroxytétrahydrofuran-3-yl]-N²-((2S)-2-{[(10H-phénothiazin-2-yloxy)acétyl]amino}-2-phényléthanoyl)-L-leucinamide :

Solide blanc. Point de fusion : 137-138 °C.

### Exemple 53 : N¹-[(3S)-2-hydroxytétrahydrofuran-3-yl]-N¹-((2S)-2-{[(10H-phénothiazin-2-yloxy)-acétyl]amino}but-3-énoyl)-L-leucinamide :

Solide rose pâle. Point de fusion : 159-161 °C.

### Exemple 54 : 2-méthyl-N-[(10H-phénothiazin-2-yloxy)acétyl]alanyl-N¹-[(3S)-2-hydroxytétrahydrofuran-3-yl]-L-leucinamide :

Solide beige. Point de fusion : 138-140 °C.

### Exemple 55 : N-[(10H-phénothiazin-2-yloxy)acétyl]glycyl-N¹-[(3S)-2-hydroxytétrahydrofuran-3-yl]-L-valinamide :

Solide beige clair. Point de fusion : 200-201 °C.

### Exemple 56 : N-[(10H-phénothiazin-2-yloxy)acétyl]glycyl-3-cyclohexyl-N¹-[(3S)-2-hydroxytétrahydrofuran-3-yl]-L-alaninamide :

Solide rose pâle. Point de fusion : 212-215 °C.

### Exemple 57 : N-[(10H-phénothiazin-2-yloxy)acétyl]glycyl-N-[(3S)-2-hydroxytétrahydrofuran-3-yl]-L-phénylalaninamide :

Solide jaune pâle. Point de fusion : 207-208 °C.

### Exemple 58 : N-[(10H-phénothiazin-2-yloxy)acétyl]glycyl-N¹-[(3S)-2-hydroxytétrahydrofuran-3-yl]-N²-isobutylglycinamide :

Solide rosé. Point de fusion : 122-124 °C.

### Exemple 59 : N-[2-méthyl-2-(10H-phénothiazin-2-yloxy)propanoyl]glycyl-N¹-[(3S)-2-méthoxytétrahydrofuran-3-yl]-L-leucinamide :

### 59.1) 2-méthyl-2-(10H-phénothiazin-2-yloxy)propanoate de tert-butyle :

A une solution de 1 g (4,6 mmol) de l'intermédiaire 21.1 dans 10 ml de DMF, on ajoute 1,93 g (3 éq.) de K₂CO₃. Le mélange réactionnel est chauffé à 60 °C avant l'addition de 1,73 ml (2 éq.) de 2-bromoisobutyrate de tertiobutyle. L'ensemble est ensuite porté à 110 °C et l'agitation est maintenue à cette température pendant 6 heures. Après retour à 20 °C, le mélange est versé dans 100 ml d'eau et le produit est extrait à l'aide de 2 fois 100 ml d'acétate d'éthyle. La solution organique est finalement lavée par 100 ml de saumure, séchée sur sulfate de sodium, filtrée et concentrée à sec sous vide. Le résidu d'évaporation est purifié sur une colonne de silice, éluant : acétate d'éthyle/heptane (1/9). On obtient 450 mg d'un solide rose pâle avec un rendement de 28%. Point de fusion : 138-140 °C.

### 59.2) acide 2-méthyl-2-(10H phénothiazin-2-yloxy)propanoïque :

Le protocole expérimental utilisé est le même que celui décrit pour l'intermédiaire 21.3, l'intermédiaire 59.1 remplaçant l'intermédiaire 21.2. On obtient 254 mg d'un solide violet avec un rendement de 67%. Point de fusion : 177-180 °C.

### 59.3) N-[2-méthyl-2-(10H-phénothiazin-2-yloxy)propanoyl]glycyl-N¹-[(3S)-2-méthoxytétrahydrofuran-3 yl]-L-leucinamide :

La stratégie de synthèse utilisée est la même que celle décrite pour la synthèse du composé de l'exemple 22, en utilisant la glycyl-N¹-[(3S)-2-méthoxytétrahydrofuran-3-yl]-L-leucinamide (préparée de manière analogue à l'intermédiaire 1.6) et de l'intermédiaire 59.2. Solide jaune pâle. Point de fusion : 100-104 °C.

### Exemple 60 : N-[2-méthyl-2-(10H-phénothiazin-2-yloxy)propanoyl]glycyl-N¹-[(3S)-2-hydroxytétrahydrofuran-3-yl]-L-leucinamide :

Le protocole expérimental est le même que celui décrit pour le composé de l'exemple 2, en partant du composé de l'exemple 59 au lieu du composé de l'exemple 1. On obtient un solide beige. Point de fusion : 127-128 °C.

### Exemple 61 : N-(10,11-dihydro-5H-dibenzo[b,f]azépin-3-ylcarbonyl)-L-leucyl-N¹-[(3S)-2-méthoxytétrahydrofuran-3-yl]-L-leucinamide :

### 61.1) acide 5-acétyl-10,11-dihydro-5H-dibenzo[b,f]azepine-3-carboxylique :

Le protocole expérimental utilisé est le même que celui décrit pour la synthèse de l'intermédiaire 1.10, en utilisant la 1-(5-acétyl-10,11-dihydro-5H-dibenzo[b]azépin-3-yl)ethanone *(*J. Chem. Soc. 1973, 859-863) comme produit de départ. On obtient un solide jaune pâle avec un rendement de 62%. Point de fusion : 189-189,5 °C.

### 61.2) acide 10,11-dihydro-5H-dibenzo[b,f]azépine-3-carboxylique :

Un mélange de 3,06 g (10,88 mmol) de l'intermédiaire 61.1 et de 3 g (45 mmol) de KOH dans 35 ml d'éthanol est chauffé à reflux pendant 15 heures. Après refroidissement à 0°C, le milieu réactionnel est acidifié à l'aide d'HCl 2*N* jusqu'à pH = 1. Le mélange est ensuite extrait à l'aide d'acétate d'éthyle puis la solution organique est lavée avec de l'eau, séchée sur sulfate de sodium, filtrée et finalement concentrée à sec. On obtient une huile brune. La spectrométrie de masse indique 20% du composé attendu et 70% de produit acétylé (intermédiaire 61.1). Le mélange est utilisé tel quel dans l'étape suivante.

### 61.3) N-(10,11-dihydro-5H-dibenzo[b,f] azepin-3-ylcarbonyl)-L-leucyl-N¹-[(3S)-2-méthoxytétrahydrofuran-3 yl]-L-leucinamide :

Ce composé a été préparé selon la même stratégie de synthèse que celle décrite pour le composé de l'exemple 1, en utilisant les intermédiaires 1.6 et 61.2 comme produits de départ.

### Exemple 62 : N-(10,11-dihydro-5H-dibenzo[b,f]azépin-3-ylcarbonyl)-L-leueyl-N¹-[(3S)-2-hydroxytétrahydrofuran-3-yl]-L-leucinamide :

Le protocole expérimental est le même que celui décrit pour le composé de l'exemple 2 en utilisant le composé de l'exemple 61 comme produit de départ. On obtient un solide beige clair. Point de fusion : 142-143 °C.

### Exemple 63 : N-[(5-acétyl-10,11-dihydro-5H-dibenzo[b,f]azepin-3-yl)carbonyl]-L-leucyl-N¹-[(3S)-2-méthoxytétrahydrofuran-3-yl]-L-leucinamide :

Ce composé a été préparé selon la même stratégie de synthèse que celle décrite pour le composé de l'exemple 1, en utilisant les intermédiaires 1.6 et 61.1 comme produits de départ.

### Exemple 64 : 2-méthyl-N-[(10H-phénothiazin-2-yloxy)acétyl]alanyl-N¹-[(3S)-2-hydroxytétrahydrofuran-3-yl]-L-leucinamide :

Le protocole expérimental est le même que celui décrit pour l'exemple 2, le composé de l'exemple 63 remplaçant le composé de l'exemple 1. On obtient un solide blanc. Point de fusion : 166-167 °C.

### Etude pharmacologique des composés de l'invention :

### Etude des effets sur la Calpaïne I humaine

Le test consiste à mesurer l'activité de l'enzyme (enzyme purifiée à partir d'érythrocytes humains) qui est incubée dans un tampon en présence d'un substrat peptidique couplé à un fluorochrome (amino-méthylcourmarine, AMC) et du calcium. L'enzyme activée par le calcium, protéolyse le substrat et libère le fragment AMC. L'AMC libéré fluoresce à 460 nm sous une excitation à 380 nm. L' activité de l'enzyme est donc proportionnelle à la quantité de fluorescence c'est à dire de fragment AMC libre. La fluorescence (380/460 nm) est mesuré à l'aide d'un fluorimètre multipuits (Victor 2, Wallac).

Le dosage se fait en micro-plaques 96 puits à fond transparent et paroi noires dans lesquels sont distribués 10 µl par puits de substance à tester dans du DMSO 10% , 45 µl de mélange réactionnel contenant la calpaïne 1 humaine à 2,2 U/ml (Calbiochem, ref: 208713), le substrat Suc Leu Tyr-AMC (Bachem, ref: I-1355) à 1,1 mM dans du tampon (Tris-HCl 110 mM; NaCI 110 mM ; EDTA 2,2 mM; EGTA 2,2 mM ; mercaptoéthanol 1,1 mM). La réaction est initiée en ajoutant 45 µl de CaCl₂ 22 mM. Pour déterminer le bruit de fond, des puits témoins sans calcium sont ajoutés sur la plaque (10 µL DMSO 10 % + 45 µl de tampon avec l'enzyme et le substrat + 45 µl H₂O). Pour déterminer l'activité totale de l'enzyme des puits témoins sans produit sont ajoutés sur la plaque (10 µL DMSO 10 % + 45 µl de tampon avec l'enzyme et le susbtrat + 45 -µl de CaCl₂ 22 mM ). Chaque concentration des produits (0,1 nM à 10 µM) est testée en double. Les plaques sont agitées et l'incubation a lieu dans l'obscurité pendant une heure à 25° C. La fluorescence est lue à 380/460 nm à l'aide du fluorimètre.

Les composés des exemples 2, 7 à 12, 16 à 18, 20, 40 à 53, 55 à 57, 60, 62 et 64 ont une CI₅₀ inférieure ou égale à 5 µM à ce test.

### Etude des effets in situ sur l'activité calpaïne dans des cellules gliales de rat (C6)

Les cellules gliales de rat C6 sont ensemencées à 25 000 cellules par puits dans des plaques à 96 puits dans du DMEM 10 % FBS. Le lendemain, les cellules qui ont adhéré sont lavées 3 fois dans du milieu DMEM sans sérum et 40 mM Hepes. Cent microlitres de l'inhibiteur de calpaïne sont déposés dans les puits. Après une heure d'incubation à 37 °C sous une atmosphère de 5 % de CO₂, 10 µl contenant le substrat fluorescent de la calpaïne (Suc-Leu-Tyr-AMC) et la maitotoxine (Sigma, ref : M-9159), pour obtenir une concentration finale dans le puits de 100 µM et de 1 nM respectivement, sont rajoutés.

Pour déterminer l'activité totale de l'enzyme cellulaire, des puits sans produit sont ajoutés sur la plaque (100 µl DMSO 100ème plus 10 µl de MTX et substrat). Les bruits de fond sont déterminés en rajoutant des puits contrôles sans MTX. Chaque concentration d'inhibiteur (0,01 µM à 100 µM) est testée en triplicats. Les plaques sont agitées, la fluorescence est lue à 380/460 nm à l'aide du Victor à T zéro. L'incubation a lieu pendant une heure trente pour les cellules C6 à 30° C à l'obscurité.

Les composés des exemples 8, 9, 11, 16, 20, 40, 43 et 47 à 53 ont une CI₅₀ inférieure ou égale à 10 µM à ce test.

### Etude des effets sur la peroxydation lipidique du cortex cérébral de rat

L'activité inhibitrice des produits de l'invention est déterminée par la mesure de leurs effets sur le degré de peroxydation lipidique, déterminée par la concentration en malondialdéhyde (MDA). Le MDA produit par la peroxydation des acides gras insaturés est un bon indice de la péroxidation lipidique (H Esterbauer and KH Cheeseman, *Meth. Enzymol.* (1990), **186**, 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50 000 g pendant 10 minutes à 4° C. Le culot est conservé à -80° C. Le jour de l'expérience, le culot est remis en suspension à la concentration de 1 g / 15 ml et centrifugé à 515 g pendant 10 minutes à 4° C. Le surnageant est utilisé immédiatement pour la détermination de la peroxydation lipidique. L'homogénat de cortex cérébral de rat (500 µl) est incubé à 37° C pendant 15 minutes en présence des composés à tester ou du solvant (10 µl). La réaction de peroxydation lipidique est initiée par l'ajout de 50 µl de FeCl₂ à 1 mM, d'EDTA à 1 mM et d'acide ascorbique à 4 mM. Après 30 minutes d'incubation à 37° C, la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll., European J. Pharmacol. (1995), 285, 203-206).

Les composés des exemples 2, 7 à 9, 11, 12, 16, 17, 20, 40 à 45, 56, 57, 60 et 62 ont une CI₅₀ inférieure ou égale à 5 µM à ce test.

## Revendications

1. Composé de formule générale **(I)** 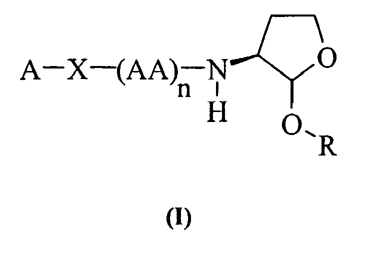 dans laquelle :
A représente le radical 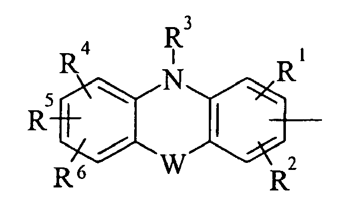 dans lequel
R¹, R², R⁴, R⁵ et R⁶ représentent, indépendamment, un atome d'hydrogène, un atome halogène, le groupe OH, un radical alkyle, alkoxy, cyano, nitro ou NR⁷R⁸,
R⁷ et R⁸ représentent, indépendamment, un atome d'hydrogène, un radical alkyle ou un groupe -COR⁹,
R⁹ représente un atome d'hydrogène, un radical alkyle ou alkoxy,
R³ représente un atome d'hydrogène, un radical alkyle ou un groupe -COR¹⁰,
R¹⁰ représente un atome d'hydrogène ou un radical alkyle ou alkoxy, et
W représente une liaison ou un radical -CH₂-CH₂-, -CH=CH-, -O-, -S- ou -NR¹¹- dans lequel R¹¹ représente un atome d'hydrogène ou un radical alkyle ;
X représente -CO-, -Y-CO-, -O-Y-CO- ou -NR¹²-Y-CO-,
Y représente un radical alkylène ou haloalkylène,
R¹² représente un atome d'hydrogène, un radical alkyle ou un groupe -COR¹³,
R¹³ représente un atome d'hydrogène, un radical alkyle, haloalkyle ou alkoxy,
AA représente, à chaque fois qu'il intervient, un aminoacide naturel, un aminoacide naturel dont la chaîne latérale qui porte une fonction chimique réactive (telle qu'acide carboxylique, amine, alcool ou thiol) est protégée sous forme d'ester d'alkyle ou d'aralkyle (pour les fonctions acide), de carbamate d'alkyle, d'aralkyle ou bien de carboxamide d'alkyle ou d'aralkyle (pour les fonctions amine), sous forme d'éther d'alkyle ou d'aralkyle ou de thioéther d'alkyle ou d'aralkyle ou bien sous forme d'ester d'alkyle ou d'aralkyle (pour les fonctions alcool et thiol) ou enfin un aminoacide de formule générale -NR¹⁴-(CH₂)ₚ-CR¹⁵R¹⁶-CO- dans laquelle p représente 0 ou 1, R¹⁴ représente un atome d'hydrogène ou un radical alkyle, R¹⁵ représente un atome d'hydrogène ou un radical alkyle et R¹⁶ un atome d'hydrogène, un radical alkyle, haloalkyle, phényle, cycloalkyle, cycloalkylalkyle ou alkényle,
ou bien R¹⁵ et R¹⁶ formant avec l'atome de carbone auquel ils sont attachés un carbocycle saturé de 3 à 7 atomes de carbone (et de préférence de 3 à 6 atomes de carbone),
un groupe -(AA)₂- pouvant aussi représenter un carbapeptide de formule générale -NR¹⁷-(CH₂)₃-CH(R¹⁸)-CO- dans laquelle R¹⁷ représente un atome d'hydrogène ou un radical alkyle et R¹⁸ représente un atome d'hydrogène ou un radical alkyle ;
n représente 2 ou 3 ; et enfin
R représente un atome d'hydrogène ou un radical alkyle ou -CO-R¹⁹ dans lequel R¹⁹ représente un radical alkyle ;
ou sel d'un tel composé.

2. Composé de formule générale **(I)** selon la revendication 1, **caractérisé en ce que** :
❖ R¹, R², R⁴, R⁵ et R⁶ représentent, indépendamment, un atome d'hydrogène, un atome halogène ou un radical alkyle, alkoxy ou NR⁷R⁸ ;
❖ R³ représente un atome d'hydrogène, un radical méthyle ou un radical -COR⁹ dans lequel R⁹ représente un radical méthyle ou *tert*-butoxy ;
❖ W représente une liaison ou un radical -CH₂-CH₂-, -CH=CH-, -O- ou -S- ;
❖ X représente -CO-, -Y-CO- ou -O-Y-CO- ;
❖ -(AA)ₙ- contient des aminoacides choisis indépendamment parmi le groupe constitué par les aminoacides naturels, la 3-méthylvaline, la norvaline, la phénylglycine, la vinylglycine et l'acide 2-aminobutyrique ;
❖ n représente 2 ; et
❖ R représente un atome d'hydrogène ou un radical méthyle ;
ou sel d'un tel composé.

3. Composé de formule générale (I) selon la revendication 1, **caractérisé en ce que** :
❖ R¹, R², R⁴, R⁵ et R⁶ représentent, indépendamment, un atome d'hydrogène ou un radical alkyle ou alkoxy ;
❖ R³ représente un atome d'hydrogène ou un radical méthyle ;
❖ W représente -S- ;
❖ X représente -Y-CO- ou -O-Y-CO- ;
❖ -(AA)ₙ- représente un -(AA²)-(AA¹)- tel que AA¹ représente Leu et AA² représente un aminoacide choisi parmi le groupe constitué par les aminoacides naturels, la 3-méthylvaline, la norvaline, la phénylglycine, la vinylglycine et l'acide 2-aminobutyrique ;
❖ R représente un atome d'hydrogène ;
ou sel d'un tel composé.

4. Composé de formule générale **(I)** selon la revendication 1, **caractérisé en ce qu'**il est choisi parmi les composés suivants :
- *N*-(10H-phénothiazin-2-ylcarbonyl)-L-leucyl-L-leucyl-*N*¹-[(3S)-2-méthoxytétrahydrofuran-3-yl]-L-leucinamide ;
- *N*-(10H-phénothiazin-2-ylcarbonyl)-L-leucyl-L-leucyl-*N*¹-[(3S)-2-hydroxytétrahydrofuran-3-yl]-L-leucinamide ;
- *N*-(10H-phénothiazin-2-ylcarbonyl)glycyl-*N¹*-[(3S)-2-méthoxytétrahydrofuran-3-yl]-L-leucinamide ;
- *N*-(10H-phénothiazin-2-ylcarbonyl)leucyl-*N*¹-[(3S)-2-méthoxytétrahydrofuran-3-yl]-L-leucinamide ;
- *N*⁶-[(benzyloxy)carbonyl]-*N*²-(10H-phénothiazin-2-ylcarbonyl)lysyl-*N*¹-[(3S)-2-méthoxytétrahydrofuran-3-yl]-L-leucinamide ;
- 1-(10H-phénothiazin-2-ylcarbonyl)-L-prolyl-*N¹*-[(3S)-2-méthoxytétrahydrofuran-3-yl]-L-leucinamide ;
- *N*-(10H-phénothiazin-2-ylcarbonyl)glycyl-*N¹*-[(3S)-2-hydroxytétrahydrofuran-3-yl]-L-leucinamide ;
- *N*-(10H-phénothiazin-2-ylcarbonyl)leucyl-*N¹*-[(3S)-2-hydroxytétrahydrofuran-3-yl]-L-leucinamide ;
- *N⁶*-[(benzyloxy)carbonyl]-*N²*-(10H-phénothiazin-2-ylcarbonyl)lysyl-*N¹*-[(35)-2-hydroxytétrahydrofuran-3-yl]-L-leucinamide ;
- 1-(10H-phénothiazin-2-ylcarbonyl)-L-prolyl-*N¹*-[(3S)-2-hydroxytétrahydrofuran-3-yl]-L-leucinamide ;
- *N*-(10H-phénothiazin-2-ylcarbonyl)leucyl-*N¹*-[(3S)-2-(acétyloxy)-tétrahydrofuran-3-yl]-L-leucinamide ;
- *N²*-(10H-phénothiazin-2-ylcarbonyl)lysyl-*N¹*-[(3 S)-2-hydroxytétrahydrofuran-3-yl]-L-leucinamide ;
- *N*-(10H-phénothiazin-2-ylacétyl)-L-leucyl-*N¹*-[(3S)-2-méthoxytétrahydrofuran-3-yl]-L-leucinamide ;
- *O*-(tert-butyl)-*N*-(10H-phénothiazin-2-ylacétyl)-L-séryl-*N*¹-[(3S)-2-méthoxytétrahydrofuran-3-yl]-L-leucinamide ;
- *N*-(10H-phénothiazin-2-ylacétyl)-L-alanyl-3-cyclohexyl-*N*¹-[(3S)-2-méthoxytétrahydrofuran-3-yl]-L-alaninamide ;
- *N*-(10H-phénothiazin-2-ylacétyl)-L-leucyl-*N*¹-[(3S)-2-hydroxytétrahydrofuran-3-yl]-L-leucinamide ;
- *O*-(tert-butyl)-*N*-(10H-phénothiazin-2-ylacétyl)-L-séryl-*N*¹-[(3S)-2-hydroxytétrahydrofuran-3-yl]-L-leucinamide ;
- *N*-(10H-phénothiazin-2-ylacétyl)-L-alanyl-3-cyclohexyl-*N*¹-[(3S)-2-hydroxytétrahydrofuran-3-yl]-L-alaninamide ;
- *N*-[3-(10H-phénothiazin-2-yl)propanoyl]-L-leucyl-*N¹*-[(3S)-2-méthoxytétrahydrofuran-3-yl]-L-leucinamide ;
- *N*-[3-(10H-phénothiazin-2-yl)propanoyl]-L-leucyl-*N¹*-[(3S)-2-hydroxytétrahydrofuran-3-yl]-L-leucinamide ;
- *N*-[(10H-phénothiazin-2-yloxy)acétyl]-L-leucyl-*N¹*-[(3S)-2-méthoxytétrahydrofuran-3-yl]-L-leucinamide ;
- *N*-[(10H-phénothiazin-2-yloxy)acétyl]-glycyl-*N¹*-[(3S)-2-méthoxytétrahydrofuran-3-yl]-L-leucinamide ;
- *N*-[(10H-phénothiazin-2-yloxy)acétyl]-L-alanyl-*N¹*-[(3S)-2-méthoxytétrahydrofuran-3-yl]-L-leucinamide ;
- *N*-[(10H-phénothiazin-2-yloxy)acétyl]-L-valyl-*N¹*-[(3S)-2-méthoxytétrahydrofuran-3-yl]-L-leucinamide ;
- *N*-[(10H-phénothiazin-2-yloxy)acétyl]-β-alanyl-*N¹*-[(3S)-2-méthoxytétrahydrofuran-3-yl]-L-leucinan>ide ;
- *N*-méthyl-*N*-[(10H-phénothiazin-2-yloxy)acétyl]glycyl-*N¹*-[(35)-2-méthoxytétrahydrofuran-3-yl]-L-leucinamide ;
- *N*-[(10H-phénothiazin-2-yloxy)acétyl]-D-valyl-*N¹*-[(3S)-2-méthoxytétrahydrofuran-3-yl]-L-leucinamide ;
- 3-méthyl-*N*-[(10H-phénothiazin-2-yloxy)acétyl]-L-valyl-*N¹*-[(3S)-2-méthoxytétrahydrofuran-3-yl]-L-leucinamide ;
- *N¹*-[(3S)-2-méthoxytétrahydrofuran-3-yl]-*N²*-((2S)-2-{[(10H-phénothiazin-2-yloxy)-acétyl]amino}butanoyl)-L-leucinamide ;
- *N*-[(10H-phénothiazin-2-yloxy)acétyl]-L-norvalyl-*N¹*-[(3S)-2-méthoxytétrahydrofuran-3-yl]-L-leucinamide ;
- *N*-[(10H-phénothiazin-2-yloxy)acétyl]-L-séryl-*N¹*-[(3S)-2-méthoxytétrahydrofuran-3-yl]-L-leucinamide ;
- *N*-[(10H-phénothiazin-2-yloxy)acétyl]-L-thréonyl-*N¹*-[(3S)-2-méthoxytétrahydrofuran-3-yl]-L-leucinamide ;
- *N¹*-[(3S)-2-méthoxytétrahydrofuran-3-yl]-*N²*-((2S)-2- {[(10H-phénothiazin-2-yloxy)acétyl]amino}-2-phényléthanoyl)-L-leucinamide ;
- *N¹*-[(3S)-2-méthoxytétrahydrofuran-3-yl]-*N²*-((2S)-2-{[(1H-phénothiazin-2-yloxy)acétyl]amino}but-3-enoyl)-L-leucinamide ;
- 2-méthyl-*N*-[(10H-phénothiazin-2-yloxy)acétyl]alanyl-*N¹*-[(3S)-2-méthoxytétrahydrofuran-3-yl]-L-leucinamide ;
- *N*-[(10H-phénothiazin-2-yloxy)acétyl]-glycyl-*N¹*-[(3S)-2-méthoxytétrahydrofuran-3-yl]-L-valinamide ;
- *N*-[(10H-phénothiazin-2-yloxy)acétyl]-glycyl-3-cyclohexyl-*N¹*-[(3S)-2-méthoxytétrahydrofuran-3-yl]-L-alaninamide ;
- *N*-[(10H-phénothiazin-2-yloxy)acétyl]-glycyl-*N*-[(3S)-2-méthoxytétrahydrofuran-3-yl]-L-phénylalaninamide ;
- *N*-[(10H-phénothiazin-2-yloxy)acétyl]glycyl-*N²*-isobutyl-*N¹*-[(3S)-2-méthoxytétrahydrofuran-3-yl]glycinamide ;
- *N*-[(10H-phénothiazin-2-yloxy)acétyl]-L-leucyl-*N*¹-[(3S)-2-hydroxytétrahydrofuran-3-yl]-L-leucinamide ;
- *N*-[(10H-phénothiazin-2-yloxy)acétyl]-glycyl-*N*¹-[(3S)-2-hydroxytétrahydrofuran-3-yl]-L-leucinamide ;
- *N*-[(10H-phénothiazin-2-yloxy)acétyl]-L-alanyl-*N*¹-[(3S)-2-hydroxytétrahydrofuran-3-yl]-L-leucinamide ;
- *N*-[(10H-1-phénothiazin-2-yloxy)acétyl]-L-valyl-*N*¹-[(3S)-2-hydroxytétrahydrofuran-3-yl]-L-leucinamide ;
- *N-*[(10H-phénothiazin-2-yloxy)acétyl]-β-alanyl-*N*¹-[(3S)-2-hydroxytétrahydrofuran-3-yl]-L-leucinamide ;
- *N*-méthyl-*N*-[(10H-phénothiazin-2-yloxy)acétyl]glycyl-*N*¹-[(3S)-2-hydroxytétrahydrofuran-3-yl]-L-leucinamide ;
- *N*-[(10H-phénothiazin-2-yloxy)acétyl]-D-valyl-*N¹*-[(3S)-2-hydroxytétrahydrofuran-3-yl]-L-leucinamide ;
- 3-méthyl-*N*-[(10H-phénothiazin-2-yloxy)acétyl]-L-valyl-*N*¹-[(3S)-2-hydroxytétrahydrofuran-3-yl]-L-leucinamide ;
- *N*¹-[(3S)-2-hydroxytétrahydrofuran-3-yl]-*N²*-((2S)-2-{([(10H-phénothiazin-2-yloxy)acétyl]amino}butanoyl)-L-leucinamide ;
- *N*-[(10H-phénothiazin-2-yloxy)acétyl]-L-norvalyl-*N*¹-[(3S)-2-hydroxytétrahydrofuran-3-yl]-L-leucinamide ;
- *N-[*(10H-phénothiazin-2-yloxy)acétyl]-L-séryl-*N*¹-[(3S)-2-hydroxytétrahydrofuran-3-yl]-L-leucinamide ;
- *N*-[(10H-phénothiazin-2-yloxy)acétyl]-L-thréonyl-*N*¹-[(3S)-2-hydroxytétrahydrofuran-3-yl]-L-leucinamide ;
- *N*¹-[(3S)-2-hydroxytétrahydrofuran-3-yl]-*N*¹-((2S)-2-{[(10H-phénothiazin-2-yloxy)acétyl]amino}-2-phényléthanoyl)-L-leucinamide ;
- *N¹-[(3*S)-2-hydroxytétrahydrofuran-3-yl]-*N*²-((2S)-2-{[(10H-phénothiazin-2-yloxy)-acétyl]amino}but-3-énoyl)-L-leucinamide ;
- 2-méthyl-*N*-[(10H-phénothiazin-2-yloxy)acétyl]alanyl. *N*¹-[(3S)-2-hydroxytétrahydrofuran-3-yl]-L-leucinamide ;
- *N*-[(10H-phénothiazin-2-yloxy)acétyl]glycyl-*N*¹-[(3S)-2-hydroxytétrahydrofuran-3-yl]-L-valinamide ;
- *N*-[(10H-phénothiazin-2-yloxy)acétyl]glycyl-3-cyclohexyl-*N*¹-[(3S)-2-hydroxytétrahydrofuran-3-yl]-L-alaninamide ;
- *N*-[(10H-phénothiazin-2-yloxy)acétyl]glycyl-*N-*[(3S)-2-hydroxytétrahydrofuran-3-yl]-L-phénylalaninamide ;
- *N*-[(10H-phénothiazin-2-yloxy)acétyl]glycyl-*N*¹-[(3S)-2-hydroxytétrahydrofuran-3-yl]-*N²*-isobutylglycinamide ;
- *N*-[2-méthyl-2-(10H-phénothiazin-2-yloxy)propanoyl]glycyl-*N¹*-[(3S)-2-méthoxytétrahydrofuran-3-yl]-L-leucinamide ;
- *N*-[2-méthyl-2-(10H-phénothiazin-2-yloxy)propanoyl]glycyl-*N*¹-[(3S)-2-hydroxytétrahydrofuran-3-yl]-L-leucinamide ;
- *N*-(10,11-dihydro-5H-dibenzo[b,f]azépin-3-ylcarbonyl)-L-leucyl. *N*¹-[(3S)-2-méthoxytétrahydrofuran-3-yl]-L-leucinamide ;
- *N*-(10,11-dihydro-5H-dibenzo[b,f]azépin-3-ylcarbonyl)-L-leucyl-*N¹-*[(3S)-2-hydroxytétrahydrofuran-3-yl]-L-leucinamide ;
- *N*- [(5-acétyl-10,11-dihydro-5H-dibenzo[b,f]azepin-3-yl)carbonyl]-L-leucyl-*N*¹-[(3S)-2-méthoxytétrahydrofuran-3-yl]-L-leucinamide ;
- 2-méthyl-*N*-[(10H-phénothiazin-2-yloxy)acétyl]alanyl-*N¹*-[(3S)-2-hydroxytétrahydrofuran-3-yl]-L-leucinamide ;
ou sel d'un de ces composés.

5. A titre de médicament, un composé de formule générale **(I)** selon la revendication 1 ou un sel pharmaceutiquement acceptable d'un tel composé.

6. Composition pharmaceutique comprenant, à titre de principe actif, un composé de formule générale **(I)** selon la revendication 1 ou un sel pharmaceutiquement acceptable d'un tel composé et au moins un excipient pharmaceutiquement acceptable.

7. Utilisation d'un composé de formule générale **(I)** selon la revendication 1 ou un sel pharmaceutiquement acceptable d'un tel composé pour préparer un médicament destiné à inhiber les calpaïnes.

8. Utilisation d'un composé de formule générale **(I)** selon la revendication 1 ou un sel pharmaceutiquement acceptable d'un tel composé pour préparer un médicament destiné à inhiber la peroxydation lipidique.

9. Utilisation d'un composé de formule générale **(I)** selon la revendication 1 ou un sel pharmaceutiquement acceptable d'un tel composé pour préparer un médicament destiné à inhiber les calpaïnes et la peroxydation lipidique.

10. Utilisation d'un composé de formule générale **(I)** selon la revendication 1 ou un sel pharmaceutiquement acceptable d'un tel composé pour préparer un médicament destiné à traiter les désordres et maladies choisis parmi le groupe constitué par les maladies inflammatoires et immunologiques, les maladies cardio-vasculaires et cérébro-vasculaires, les troubles du système nerveux central ou périphérique, l'ostéoporose, les dystrophies musculaires, les maladies prolifératives, la cataracte, les réactions de rejet suite à des transplantations d'organes et les maladies auto-immunes et virales.

## Claims

1. Compound of general formula (I) 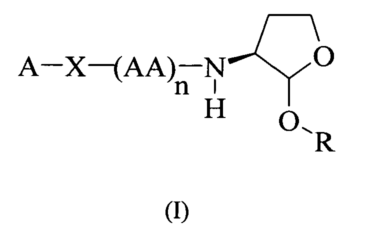 in which:
A represents the 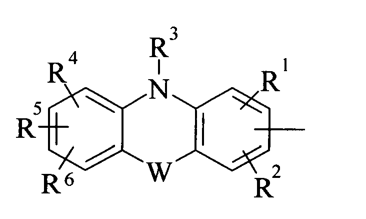 radical, in which
R¹, R², R⁴, R⁵ and R⁶ represent, independently, a hydrogen atom, a halogen atom, the OH group, an alkyl, alkoxy, cyano, nitro or NR⁷R⁸ radical,
R⁷ and R⁸ represent, independently, a hydrogen atom, an alkyl radical or a -COR⁹ group,
R⁹ represents a hydrogen atom, an alkyl or alkoxy radical,
R³ represents a hydrogen atom, an alkyl radical or a -COR¹⁰ group,
R¹⁰ represents a hydrogen atom or an alkyl or alkoxy radical, and
W represents a bond or a -CH₂-CH₂-, -CH=CH-, -O-, -S- or -NR¹¹- radical in which R¹¹ represents a hydrogen atom or an alkyl radical;
X represents -CO-, -Y-CO-, -O-Y-CO- or -NR¹²-Y-CO,
Y represents an alkylene or haloalkylene radical,
R¹² represents a hydrogen atom, an alkyl radical or a -COR¹³ group,
R¹³ represents a hydrogen atom, an alkyl, haloalkyl or alkoxy radical,
AA represents, each time that it occurs, a natural amino acid, a natural amino acid the side chain of which, which carries a reactive chemical function (such as carboxylic acid, amine, alcohol or thiol), is protected in the form of alkyl or aralkyl ester (for the acid functions), alkyl or aralkyl carbamate or alkyl or aralkyl carboxamide (for the amine functions), in the form of alkyl or aralkyl ether or alkyl or aralkyl thioether or in the form of alkyl or aralkyl ester (for the alcohol and thiol functions) or finally an amino acid of general formula NR¹⁴-(CH₂)ₚ CR¹⁵R¹⁶-CO- in which p represents 0 or 1, R¹⁴ represents a hydrogen atom or an alkyl radical, R¹⁵ represents a hydrogen atom or an alkyl radical and R¹⁶ a hydrogen atom, an alkyl, haloalkyl, phenyl, cycloalkyl, cycloalkylalkyl or alkenyl radical,
or R¹⁵ and R¹⁶ forming with the carbon atom to which they are attached a saturated carbocycle with 3 to 7 carbon atoms (and preferably with 3 to 6 carbon atoms), an -(AA)₂- group also being able to represent a carbapeptide of general formula -NR¹⁷ -(CH₂)₃-CH(R¹⁸ )-CO- in which R¹⁷ represents a hydrogen atom or an alkyl radical and R¹⁸ represents a hydrogen atom or an alkyl radical;
n represents 2 or 3; and finally
R represents a hydrogen atom or an alkyl or -CO-R¹⁹ radical in which R¹⁹ represents an alkyl radical;
or salt of such a compound.

2. Compound of general formula (I) according to claim 1, **characterized in that**:
❖ R¹, R², R⁴, R⁵ and R⁶ represent, independently, a hydrogen atom, a halogen atom or an alkyl, alkoxy or NR⁷ R⁸ radical;
❖ R³ represents a hydrogen atom, a methyl radical or a -COR⁹ radical in which R⁹ represents a methyl or tert-butoxy radical;
❖ W represents a bond or a -CH₂-CH₂-, -CH=CH-, -O- or -S-;
❖ X represents -CO-, -Y-CO- or -O-Y-CO-;
❖ -(AA)ₙ- contains amino acids chosen independently from the group constituted by the natural amino acids, 3-methylvaline, norvaline, phenylglycine, vinylglycine and 2-aminobutyric acid;
❖ n represents 2; and
❖ R represents a hydrogen atom or a methyl radical;
or salt of such a compound.

3. Compound of general formula **(I)** according to claim 1, **characterized in that**:
❖ R¹, R², R⁴, R⁵ and R⁶ represent, independently, a hydrogen atom or an alkyl or alkoxy radical;
R³ represents a hydrogen atom or a methyl radical;
❖ W represents -S-;
❖ X represents -Y-CO- or -O-Y-CO-;
❖ -(AA)ₙ- represents an -(AA²)-(AA¹)- such that AA¹ represents Leu and AA² represents an amino acid chosen from the group constituted by the natural amino acids, 3-methylvaline, norvaline, phenylglycine, vinylglycine and 2-aminobutyric acid;
❖ R represents a hydrogen atom;
or salt of such a compound.

4. Compound of general formula **(I)** according to claim 1, **characterized in that** it is chosen from the following compounds:
- N-(10H-phenothiazin-2-ylcarbonyl)-L-leucyl-L-leucyl-N¹-[(3S)-2-methoxytetrahydrofuran-3-yl]-L-leucinamide;
- N-( 10H-phenothiazin-2-ylcarbonyl)-L-leucyl-L-leucyl-N¹-[(3S)-2-hydroxytetrahydrofuran-3-yl]-L-leucinamide;
- N-(10H-phenothiazin-2-ylcarbonyl)glycyl-N¹-[(3S)-2-methoxytetrahydrofuran-3-yl]-L-leucinamide;
- N-(10H-phenothiazin-2-ylcarbonyl)leucyl-N¹-[(3S)-2-methoxytetrahydrofuran-3-yl]-L-leucinamide;
- N⁶-[(benzyloxy)carbonyl]-N²-(10H-phenothiazin-2-ylcarbonyl)lysyl-N¹-[(3S)-2-methoxytetrahydrofuran-3-yl]-L-leucinamide;
- 1-(10H-phenothiazin-2-ylcarbonyl)-L-prolyl-N¹-[(3S)-2-methoxytetrahydrofuran-3-yl]-L-leucinamide;
- N-(10H-phenothiazin-2-ylcarbonyl)glycyl-N¹-[(3S)-2-hydroxytetrahydrofuran-3-yl]-L-leucinamide;
- N-(10H-phenothiazin-2-ylcarbonyl)leucyl-N¹-[(3S)-2-hydroxytetrahydrofuran-3-yl]-L-leucinamide;
- N⁶-[(benzyloxy)carbonyl]-N²-(10H-phenothiazin-2-ylcarbonyl)lysyl-N¹-[(3S)-2-hydroxytetrahydrofuran-3-yl]-L-leucinamide;
- 1-(10H-phenothiazin-2-ylcarbonyl)-L-prolyl-N¹-[(3S)-2-hydroxytetrahydrofuran-3-yl]-L-leucinamide;
- N-(10H-phenothiazin-2-ylcarbonyl)leucyl-N¹-[(3S)-2-(acetyloxy)-tetrahydrofuran-3-yl]-L-leucinamide;
- N²-(10H-phenothiazin-2-ylcarbonyl)lysyl-N¹-[(3S)-2-hydroxytetrahydrofuran-3-yl]-L-leucinamide;
- N-(10H-phenothiazin-2-ylacetyl)-L-leucyl-N¹-[(3S)-2-methoxytetrahydrofuran-3-yl]-L-leucinamide;
- O-(tert-butyl)-N-(10H-phenothiazin-2-ylacetyl)-L-seryl-N¹-[(3S)-2-methoxytetrahydrofuran-3-yl]-L-leucinamide;
- N-(10H-phenothiazin-2-ylacetyl)-L-alanyl-3-cyclohexyl-N¹[(3 S)-2-methoxytetrahydrofuran-3-yl]-L-alaninamide;
- N-(10H-phenothiazin-2-ylacetyl)-L-leucyl-N¹-[(3S)-2-hydroxytetrahydrofuran-3-yl]-L-leucinamide;
- O-(tert-butyl)-N-(10H-phenothiazin-2-ylacetyl)-L-seryl-N¹-[(3 S)-2-hydroxytetrahydrofuran-3-yl]-L-leucinamide;
- N-(10H-phenothiazin-2-ylacetyl)-L-alanyl-3 -cyclohexyl-N¹-[(3S)-2-hydroxytetrahydrofuran-3-yl]-L-alaninamide;
- N-[3-(10H-phenothiazin-2-yl)propanoyl]-L-leucyl-N¹-[(3S)-2-methoxytetrahydrofuran-3-yl]-L-leucinamide;
- N-[3-(10H-phenothiazin-2-yl)propanoyl]-L-leucyl-N¹-[(3S)-2-hydroxytetrahydrofuran-3-yl]-L-leucinamide;
- N-[(10H-phenothiazin-2-yloxy)acetyl]-L-leucyl-N¹-[(3S)-2-methoxytetrahydrofuran-3-yl]-L-leucinamide;
- N-[(10H-phenothiazin-2-yloxy)acetyl]-glycyl-N¹-[(3S)-2-methoxytetrahydrofuran-3-yl]-L-leucinamide;
- N-[(10H-phenothiazin-2-yloxy)acetyl]-L-alanyl-N¹-[(3S)-2-methoxytetrahydrofuran-3-yl]-L-leucinamide;
- N-[(10H-phenothiazin-2-yloxy)acetyl]-L-valyl-N¹-[(3S)-2-methoxytetrahydrofuran-3-yl]-L-leucinamide;
- N-[(10H-phenothiazin-2-yloxy)acetyl]-β-alanyl-N'-[(3S)-2-methoxytetrahydrofuran-3-yl]-L-leucinamide;
- N-methyl-N-[(10H-phenothiazin-2-yloxy)acetyl]glycyl-N¹-[(3S)-2-methoxytetrahydrofuran-3-yl]-L-leucinamide;
- N-[(10H-phenothiazin-2-yloxy)acetyl]-D-valyl-N¹-[(3S)-2-methoxytetrahydrofuran-3-yl]-L-leucinamide;
- 3-methyl-N-[(10H-phenothiazin-2-yloxy)acetyl]-L-valyl-N¹-[(3S)-2-methoxytetrahydrofuran-3-yl]-L-leucinamide;
- N¹-[(3S)-2-methoxytetrahydrofuran-3-yl]-N²-((2S)-2-{[(10H-phenothiazin-2-yloxy)-acetyl] amino} butanoyl)-L-leucinamide;
- N-[(10H-phenothiazin-2-yloxy)acetyl]-L-norvalyl-N¹-[(3S)-2-methoxytetrahydrofuran-3-yl]-L-leucinamide;
- N-[(10H-phenothiazin-2-yloxy)acetyl]-L-seryl-N'-[(3S)-2-methoxytetrahydrofuran-3-yl]-L-leucinamide;
- N-[(10H-phenothiazin-2-yloxy)acetyl]-L-threonyl-N¹-[(3S)-2-methoxytetrahydrofuran-3-yl]-L-leucinamide;
- N¹-[(3S)-2-methoxytetrahydrofuran-3-yl]-N²-((2S)-2-{[(10H-phenothiazin-2-yloxy)acetyl] amino}-2-phenylethanoyl)-L-leucinamide;
- N¹-[(3S)-2-methoxytetrahydrofuran-3-yl]-N²-((2S)-2-{[(10H-phenothiazin-2-yloxy)acetyl]amino}but-3-enoyl)-L-leucinamide;
- 2-methyl-N-[(10H-phenothiazin-2-yloxy)acetyl]alanyl-N¹-[(3 S)-2-methoxytetrahydrofuran-3-yl]-L-leucinamide;
- N-[(10H-phenothiazin-2-yloxy)acetyl]-glycyl-N¹-[(3S)-2-methoxytetrahydrofuran-3-yl]-L-valinamide;
- N-[(10H-phenothiazin-2-yloxy)acetyl]-glycyl-3-cyclohexyl-N¹-[(3S)-2-methoxytetrahydrofuran-3-yl]-L-alaninamide;
- N-[(10H-phenothiazin-2-yloxy)acetyl]-glycyl-N-[(3S)-2-methoxytetrahydrofuran-3-yl]-L-phenylalaninamide;
- N-[(10H-phenothiazin-2-yloxy)acetyl]glycyl-N²-isobutyl-N¹-[(3S)-2-methoxytetrahydrofuran-3-yl]glycinamide;
- N-[(10H-phenothiazin-2-yloxy)acetyl]-L-leucyl-N¹-[(3S)-2-hydroxytetrahydrofuran-3-yl]-L-leucinamide;
- *N*¹-[(10H-phenothiazin-2-yloxy)acetyl]-glycyl-*N*¹-[(3S)-2-hydroxytetrahydrofuran-3-yl]-L-leucinamide;
- N-[(10-phenothiazin-2-yloxy)acetyl]-L-alanyl-*N*¹-[(3S)-2-hydroxytetrahydrofuran-3-yl]-L-leucinamide;
- *N*-[(10H-phenothiazin-2-yloxy)acetyl]-L-valyl-*N¹*-[(3S)-2-hydroxytetrahydrofuran-3-yl]-L-leucinamide;
- N-[(10H-phenothiazin-2-yloxy)acetyl]-β-alanyl-*N¹*-[(3S)-2-hydroxytetrahydrofuran-3-yl]-L-leucinamide;
- N-methyl-N-[(10H-phenothiazin-2-yloxy)acetyl]glycyl-N¹-[(3S)-2-hydroxytetrahydrofuran-3-yl]-L-leucinamide;
- N-[(10H-phenothiazin-2-yloxy)acetyl]-D-valyl-*N¹*-[(3S)-2-hydroxytetrahydrofuran-3-yl]-L-leucinamide;
- 3-methyl-N-[(10H-phenothiazin-2-yloxy)acetyl]-L-valyl-N¹-[(3S)-2-hydroxytetrahydrofuran-3-yl]-L-leucinamide;
- N¹-[(3S)-2-hydroxytetrahydrofuran-3-yl]-N²-((2S)-2-{[(10H-phenothiazin-2-yloxy)acetyl] amino } butanoyl)-L-leucinamide;
- N-[(10H-phenothiazin-2-yloxy)acetyl]-L-norvalyl-N¹-[(3S)-2-hydroxytetrahydrofuran-3-yl]-L-leucinamide;
- N-[(10H-phenothiazin-2-yloxy)acetyl]-L-seryl-N¹-[(3S)-2-hydroxytetrahydrofuran-3-yl]-L-leucinamide;
- N- [(1 0H-phenothiazin-2-yloxy)acetyl] -L-threonyl-N¹ - [(3 S)-2-hydroxytetrahydrofuran-3-yl]-L-leucinamide;
- N¹-[(3S)-2-hydroxytetrahydrofuran-3-yl]-N² -((2S)-2-{[(1 0H-phenothiazin-2-yloxy)acetyl]amino }-2-phenylethanoyl)-L-leucinamide;
- N¹-[(3S)-2-hydroxytetrahydrofuran-3-yl]-N²-((2S)-2-{[(10H-phenothiazin-2-yloxy)-acetyl] amino } but-3 -enoyl)-L-leucinamide;
- 2-methyl-N-[(10H-phenothiazin-2-yloxy)acetyl]alanyl-N¹-[(3S)-2-hydroxytetrahydrofuran-3-yl]-L-leucinamide;
- N-[(10H-phenothiazin-2-yloxy)acetyl]glycyl-N¹-[(3S)-2-hydroxytetrahydrofuran-3-yl]-L-valinamide;
- N-[(10H-phenothiazin-2-yloxy)acetyl]glycyl-3-cyclohexyl-N¹-[(3S)-2-hydroxytetrahydrofuran-3-yl]-L-alaninamide;
- N-[(10H-phenothiazin-2-yloxy)acetyl]glycyl-N-[(3S)-2-hydroxytetrahydrofuran-3-yl]- L-phenylalaninamide;
- N-[(10H-phenothiazin-2-yloxy)acetyl]glycyl-N¹-[(35)-2-hydroxytetrahydrofuran-3-yl]-N²-isobutylglycinamide;
- N-[2-methyl-2-(1H-phenothiazin-2-yloxy)propanoyl]glycyl-N¹-[(3S)-2-methoxytetrahydrofuran-3-yl]-L-leucinamide;
- N-[2-methyl-2-(1H-phenothiazin-2-yloxy)propanoyl]glycyl-N¹-[(3S)-2-hydroxytetrahydrofuran-3-yl]-L-leucinamide;
- N-(10,11-dihydro-5H-dibenzo[b,f]azepin-3-ylcarbonyl)-L-leucyl-N¹-[(3S)-2-methoxytetrahydrofuran-3-yl]-L-leucinamide;
- N-(10,11-dihydro-5H-dibenzo[b,f]azepin-3-ylcarbonyl)-L-leucyl-N¹-[(3S)-2-hydroxytetrahydrofuran-3-yl]-L-leucinamide;
- N-[(5-acetyl-10,11-dihydro-5H-dibenzo[b,f]azepin-3-yl)carbonyl]-L-leucyl-N¹-[(3S)-2-methoxytetrahydrofuran-3-yl]-L-leucinamide;
- 2-methyl-N-[(10H-phenothiazin-2-yloxy)acetyl]alanyl-N¹-[(3S)-2-hydroxytetrahydrofuran-3-yl]-L-leucinamide;
or salt of one of these compounds.

5. As a medicament, a compound of general formula **(I)** according to claim 1 or a pharmaceutically acceptable salt of such a compound.

6. Pharmaceutical composition comprising, as active ingredient, a compound of general formula **(I)** according to claim 1 or a pharmaceutically acceptable salt of such a compound and at least one pharmaceutically acceptable excipient.

7. Use of a compound of general formula **(I)** according to claim 1 or a pharmaceutically acceptable salt of such a compound for preparing a medicament intended to inhibit calpains.

8. Use of a compound of general formula **(I)** according to claim 1 or a pharmaceutically acceptable salt of such a compound for preparing a medicament intended to inhibit lipid peroxidation.

9. Use of a compound of general formula **(I)** according to claim 1 or a pharmaceutically acceptable salt of such a compound for preparing a medicament intended to inhibit calpains and lipid peroxidation.

10. Use of a compound of general formula **(I)** according to claim 1 or a pharmaceutically acceptable salt of such a compound for preparing a medicament intended to treat disorders and diseases chosen from the group constituted by the inflammatory and immunological diseases, cardio-vascular and cerebro-vascular diseases, disorders of the central or peripheral nervous system, osteoporosis, muscular dystrophy, proliferative diseases, cataract, rejection reactions following organ transplants and autoimmune and viral diseases.

## Patentansprüche

1. Verbindung der allgemeinen Formel (I) 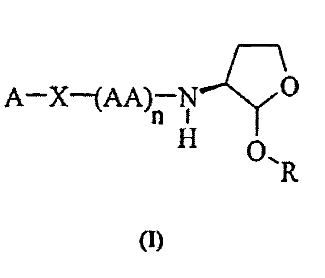 in der:
A den Rest darstellt 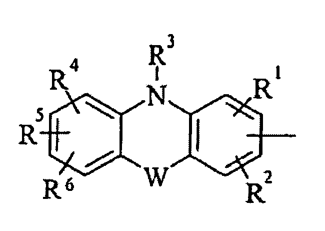 in dem
R¹, R², R⁴, R⁵und R⁶ unabhängig voneinander ein Wasserstoffatom, ein Halogenatom, die OH-Gruppe, einen Rest Alkyl, Alkoxy, Cyano, Nitro oder NR⁷R⁸ darstellen,
R⁷ und R⁸ unabhängig voneinander ein Wasserstoffatom, einen Alkylrest oder eine Gruppe -COR⁹ darstellen,
R⁹ ein Wasserstoffatom, einen Alkyl- oder Alkoxyrest darstellt,
R³ ein Wasserstoffatom, einen Alkylrest oder eine Gruppe -COR¹⁰ darstellt,
R¹⁰ ein Wasserstoffatom oder einen Alkyl- oder Alkoxyrest darstellt und
W einen Bindung oder einen Rest -CH₂-CH₂-, -CH=CH-, -O-, -S- oder -NR¹¹- darstellt, in dem R¹¹ ein Wasserstoffatom oder einen Alkylrest darstellt;
X -CO-, -Y-CO-, -O-Y-CO- oder -NR¹²-Y-CO- darstellt, Y einen Alkylen- oder Halogenalkylenrest darstellt,
R¹² ein Wasserstoffatom, einen Alkylrest oder eine Gruppe -COR¹³ darstellt,
R¹³ ein Wasserstoffatom, einen Alkyl-, Halogenalkyl-oder Alkoxyrest darstellt,
AA bei jedem Auftreten darstellt: eine natürliche Aminosäure, eine natürliche Aminosäure, deren Seitenkette, die eine reaktive chemische Funktion (wie Carbonsäure, Amin, Alkohol oder Thiol) trägt, geschützt ist in Form von Alkylester oder Aralkylester (für die Säurefunktionen), von Alkylcarbamat, Aralkylcarbamat oder Alkylcarboxamid oder Aralkylcarboxamid (für die Aminfunktionen), in Form von Alkyl- oder Aralkylether oder von Alkyl- oder Aralkylthioether oder in Form von Alkyl- oder Aralkylester (für die Alkohol- und Thiolfunktionen), oder eine Aminosäure der allgemeinen Formel -NR¹⁴-(CH₂)ₚ-CR¹⁵R¹⁶-CO-, in der p 0 oder 1 darstellt, R¹⁴ ein Wasserstoffatom oder einen Alkylrest darstellt, R¹⁵ ein Wasserstoffatom oder einen Alkylrest und R¹⁶ ein Wasserstoffatom, einen Rest Alkyl, Halogenalkyl, Phenyl, Cycloalkyl, Cycloalkylalkyl oder Alkenyl darstellt,
oder R¹⁵ und R¹⁶ mit dem Kohlenstoffatom, an das sie gebunden sind, einen gesättigten Kohlenstoffring mit 3 bis 7 Kohlenstoffatomen (und vorzugsweise 3 bis 6 Kohlenstoffatomen) bilden,
wobei eine Gruppe -(AA)₂- auch ein Carbapeptid der allgemeinen Formel -NR¹⁷-(CH₂)₃-CH(R¹⁸)-CO- darstellen kann, in der R¹⁷ ein Wasserstoffatom oder einen Alkylrest darstellt und R¹⁸ ein Wasserstoffatom oder einen Alkylrest darstellt;
n 2 oder 3 darstellt; und
R ein Wasserstoffatom oder einen Rest Alkyl oder -CO-R¹⁹ darstellt, in dem R¹⁹ einen Alkylrest darstellt;
oder ein Salz einer solchen Verbindung.

2. Verbindung der allgemeinen Formel (I) nach Anspruch 1,
**dadurch gekennzeichnet, dass**:
❖ R¹, R², R⁴, R⁵ und R⁶ unabhängig voneinander ein Wasserstoffatom, ein Halogenatom oder einen Rest Alkyl, Alkoxy oder NR⁷R⁸ darstellen;
❖ R³ ein Wasserstoffatom, einen Methylrest oder einen Rest -COR⁹ darstellt, in dem R⁹ einen Methyl- oder *tert*-Butoxyrest darstellt;
❖ W eine Bindung oder einen Rest -CH₂-CH₂-, -CH=CH-, -O- oder -S darstellt;
❖ X -CO-, -Y-CO- oder -O-Y-CO- darstellt;
❖ -(AA)ₙ- Aminosäuren enthält, die unabhängig voneinander aus der Gruppe ausgewählt sind, die aus den natürlichen Aminosäuren, 3-Methylvalin, Norvalin, Phenylglycin, Vinylglycin und 2-Aminobuttersäure besteht;
❖ n 2 darstellt; und
❖ R ein Wasserstoffatom oder einen Methylrest darstellt;
oder ein Salz einer solchen Verbindung.

3. Verbindung der allgemeinen Formel (I) nach Anspruch 1,
**dadurch gekennzeichnet, dass**:
❖ R¹, R², R⁴, R⁵ und R⁶ unabhängig voneinander ein Wasserstoffatom oder einen Alkyl- oder Alkoxyrest darstellen;
❖ R³ ein Wasserstoffatom oder einen Methylrest darstellt;
❖ W -S- darstellt;
❖ X -Y-CO- oder -O-Y-CO- darstellt;
❖ -(AA)ₙ- ein solches - (AA²) - (AA¹) - darstellt, dass AA¹ Leu darstellt und AA² eine Aminosäure darstellt, die aus der Gruppe ausgewählt ist, die aus den natürlichen Aminosäuren, 3-Methylvalin, Norvalin, Phenylglycin, Vinylglycin und 2-Aminobuttersäure besteht;
❖ R ein Wasserstoffatom darstellt;
oder ein Salz einer solchen Verbindung.

4. Verbindung der allgemeinen Formel (I) nach Anspruch 1, **dadurch gekennzeichnet, dass** sie aus den folgenden Verbindungen ausgewählt ist:
- *N*-(10H-Phenothiazin-2-ylcarbonyl)-L-leucyl-L-leucyl-*N¹*-[(3S)-2-methoxytetrahydrofuran-3-yl]-L-leucinamid;
- *N*-(10H-Phenothiazin-2-ylcarbonyl)-L-leucyl-L-leucyl-*N¹*-[(3S)-2-hydroxytetrahydrofuran-3-yl]-L-leucinamid;
- *N*(10H-Phenothiazin-2-ylcarbonyl)glycyl-*N¹*-[(3S)-2-methoxytetrahydrofuran-3-yl]-L-leucinamid;
- *N*-(10H-Phenothiazin-2-ylcarbonyl)leucyl-*N¹*-[(3S)-2-methoxytetrahydrofuran-3-yl]-L-leucinamid;
- *N⁶*-[(Benzyloxy)carbonyl]-*N²*-(10H-phenothiazin-2-ylcarbonyl)lysyl-*N¹*-[(3S)-2-methoxytetrahydrofuran-3-yl]-L-leucinamid;
- 1-(10H-Phenothiazin-2-ylcarbonyl)-L-prolyl-*N¹*-[(3S)-2-methoxytetrahydrofuran-3-yl]-L-leucinamid;
- *N*-(10H-Phenothiazin-2-ylcarbonyl)glycyl-*N*¹-[(3S)-2-hydroxytetrahydrofuran-3-yl]-L-leucinamid;
- *N*-(10H-Phenothiazin-2-ylcarbonyl)leucyl-*N*¹-[(3S)-2-hydroxytetrahydrofuran-3-yl]-L-leucinamid;
- *N⁶-*[(Benzyloxy)carbonyl]-*N*²-(10H-phenothiazin-2-ylcarbonyl)lysyl-*N¹*-[(3S)-2-hydroxytetrahydrofuran-3-yl]-L-leucinamid;
- 1-(10H-Phenothiazin-2-ylcarbonyl)-L-prolyl-*N¹*-[(3S)-2-hydroxytetrahydrofuran-3-yl]-L-leucinamid;
- *N*-(10H-Phenothiazin-2-ylcarbonyl)leucyl-*N¹*-[(3S)-2-(acetyloxy)-tetrahydrofuran-3-yl]-L-leucinamid;
- *N²-*(1H-Phenothiazin-2-ylcarbonyl)lysyl-*N¹*-[(3S)*-2-*hydroxytetrahydrofuran-3-yl]-L-leucinamid;
- *N*-(10H-Phenothiazin-2-ylacetyl)-L-leucyl-*N*¹-[(3S)-2-methoxytetrahydrofuran-3-yl]-L-leucinamid;
- O-(tert-Butyl)-*N*-(10H-phenothiazin-2-ylacetyl)-L-seryl-*N*¹-[(3S)-2-methoxytetrahydrofuran-3-yl]-L-leucinamid;
- *N*-(10H-Phenothiazin-2-ylacetyl)-L-alanyl-3-cyclohexyl-*N¹*-[(3S)-2-methoxytetrahydrofuran-3-yl]-L-alaninamid;
- *N*-(10H-Phenothiazin-2-ylacetyl)-L-leucyl-*N*¹-[(3S)-2-hydroxytetrahydrofuran-3-yl]-L-leucinamid;
- *O*-(tert-Butyl)-*N*-(10H-phenothiazin-2-ylacetyl)-L-seryl-*N*¹-[(3S)-2-hydroxytetrahydrofuran-3-yl]-L-leucinamid;
- *N*-(10H-Phenothiazin-2-ylacetyl)-L-alanyl-3-cyclohexyl-*N¹*-[(3S)-2-hydroxytetrahydrofuran-3-yl]-L-alaninamid;
- *N*-[3-(10H-Phenothiazin-2-yl)propanoyl]-L-leucyl-*N¹-*[(3S)-2-methoxytetrahydrofuran-3-yl]-L-leucinamid;
- *N*-[3-(10H-Phenothiazin-2-yl)propanoyl]-L-leucyl-*N*¹-[(3S)-2-hydroxytetrahydrofuran-3-yl]-L-leucinamid;
- *N*-[(10H-Phenothiazin-2-yloxy)acetyl]-L-leucyl-*N*¹-[(3S)-2-methoxytetrahydrofuran-3-yl]-L-leucinamid;
- *N*-[(10H-Phenothiazin-2-yloxy)acetyl]-glycyl-*N*¹-[(3S)-2-methoxytetrahydrofuran-3-yl]-L-leucinamid;
- *N*-[(10H-Phenothiazin-2-yloxy)acetyl]-L-alanyl-*N*¹-[(3S)-2-methoxytetrahydrofuran-3-yl]-L-leucinamid;
- *N*-[(10H-Phenothiazin-2-yloxy)-L-valyl-*N¹*-[(3S)-2-methoxytetrahydrofuran-3-yl]-L-leucinamid;
- *N*-[(10H-Phenothiazin-2-yloxy)acetyl]-β-alanyl-*N¹-*[(3S)-2-methoxytetrahydrofuran-3-yl]-L-leucinamid;
- *N*-Methyl-*N*-[(10H-phenothiazin-2-yloxy)acetyl]glycyl-*N¹*-[(3S)-2-methoxytetrahydrofuran-3-yl]-L-leucinamid;
- *N*-[(10H-Phenothiazin-2-yloxy)acetyl]-D-valyl-*N¹-*[(3S)-2-methoxytetrahydrofuran-3-yl]-L-leucinamid;
- 3-Methyl-*N*-[(10H-phenothiazin-2-yloxy)acetyl]-L-valyl-*N¹*-[(3S)-2-methoxytetrahydrofuran-3-yl]-L-leucinamid;
- *N¹-[*(3S)-2-Methoxytetrahydrofuran-3-yl]-*N²*-((2S)-2-{[(10H-phenothiazin-2-yloxy)-acetyl]amino}butanoyl)-L-leucinamid;
- *N*-[(10H-Phenothiazin-2-yloxy)acetyl]-L-norvalyl-N*¹-*[(3S)-2-methoxytetrahydrofuran-3-yl]-L-leucinamid;
- *N*-[(10H-Phenothiazin-2-yloxy)acetyl]-L-seryl-*N¹-*[(3S)-2-methoxytetrahydrofuran-3-yl]-L-leucinamid;
- *N*-[(10H-Phenothiazin-2-yloxy)acetyl]-L-threonyl-*N*¹-[(3S)-2-methoxytetrahydrofuran-3-yl]-L-leucinamid;
- *N¹-*[(3S)-2-Methoxytetrahydrofuran-3-yl]-*N*²-((2S)-2-{[(10H-phenothiazin-2-yloxy)acetyl]amino}-2-phenylethanoyl)-L-leucinamid;
- *N*¹-[(3S)-2-Methoxytetrahydrofuran-3-yl]*-N²-*((2S)-2-{[(10H-phenothiazin-2-yloxy)acetyl]amino}but-3-enoyl)-L-leucinamid;
- 2-Methyl-N-[(10H-phenothiazin-2-yloxy)acetyl]alanyl-*N¹-*[(3S)-2-methoxytetrahydrofuran-3-yl]-L-leucinamid;
- *N*-[(10H-Phenothiazin-2-yloxy)acetyl]-glycyl-*N¹-*[(3S)-2-methoxytetrahydrofuran-3-yl]-L-valinamid;
- *N*-[(10H-Phenothiazin-2-yloxy)acetyl]-glycyl-3-cyclohexyl-*N¹*-[(3S)-2-methoxytetrahydrofuran-3-yl]-L-alaninamid;
- *N*-[(10H-Phenothiazin-2-yloxy)acetyl]-glycyl-*N*-[(3S)-2-methoxytetrahydrofuran-3-yl]-L-phenylalaninamid;
- *N*-[(10H-Phenothiazin-2-yloxy)acetyl]glycyl-*N*²-isobutyl-*N*¹-[(3S)-2-methoxytetrahydrofuran-3-yl]glycinamid;
- *N*-[(10H-Phenothiazin-2-yloxy)acetyl]-L-leucyl-*N*¹-[(3S)-2-hydroxytetrahydrofuran-3-yl]-L-leucinamid;
- *N*-[(10H-Phenothiazin-2-yloxy)acetyl]-glycyl-*N¹-*[(3S)-2-hydroxytetrahydrofuran-3-yl]-L-leucinamid;
- *N*-[(10H-Phenothiazin-2-yloxy)acetyl]-L-alanyl-*N¹-*[(3S)-2-hydroxytetrahydrofuran-3-yl]-L-leucinamid;
- *N*-[(10H-Phenothiazin-2-yloxy)acetyl]-L-valyl-*N¹-*[(3S)-2-hydroxytetrahydrofuran-3-yl]-L-leucinamid;
- *N*-[(10H-Phenothiazin-2-yloxy)acetyl]-β-alanyl-*N¹-*[(3S)-2-hydroxytetrahydrofuran-3-yl]-L-leucinamid;
- *N*-Methyl-*N*-[(10H-phenothiazin-2-yloxy)acetyl]glycyl-*N¹*-[(3S)-2-hydroxytetrahydrofuran-3-yl]-L-leucinamid;
- *N*-[(10H-Phenothiazin-2-yloxy)acetyl]-D-valyl-*N¹-*[(3S)-2-hydroxytetrahydrofuran-3-yl]-L-leucinamid;
- 3-Methyl-*N*-[(10H-phenothiazin-2-yloxy)acetyl]-L-valyl-*N¹*-[(3S)-2-hydroxytetrahydrofuran-3-yl]-L-leucinamid;
- *N¹-*[(3S)-2-Hydroxytetrahydrofuran-3-yl]-*N*²-((2S)-2-{[(10H-phenothiazin-2-yloxy)acetyl]amino}butanoyl)-L-leucinamid;
- *N*-[(10H-Phenothiazin-2-yloxy)acetyl]-L-norvalyl-*N¹-*[(3S)-2-hydroxytetrahydrofuran-3-yl]-L-leucinamid;
- *N*-[(10H-Phenothiazin-2-yloxy)acetyl]-L-seryl-*N*¹-[(3S)-2-hydroxytetrahydrofuran-3-yl]-L-leucinamid;
- *N*-[(10H-Phenothiazin-2-yloxy)acetyl]-L-threonyl-*N¹-*[(3S)-2-hydroxytetrahydrofuran-3-yl]-L-leucinamid;
- *N¹-*[(3S)-2-Hydroxytetrahydrofuran-3-yl]-*N²*-((2S)-2-{[(10H-phenothiazin-2-yloxy)acetyl]amino}-2-phenylethanoyl)-L-leucinamid;
- *N¹-*[(3S)-2-Hydroxytetrahydrofuran-3-yl]*-N²-*((2S)-2-{[(10H-phenothiazin-2-yloxy)acetyl]amino}but-3-enoyl)-L-leucinamid;
- 2-Methyl-*N*-[(10H-phenothiazin-2-yloxy)acetyl]alanyl-*N¹-*[(3S)-2-hydroxytetrahydrofuran-3-yl]-L-leucinamid;
- *N*-[(10H-Phenothiazin-2-yloxy)acetyl]glycyl-*N¹*-[(3S)-2-hydroxytetrahydrofuran-3-yl]-L-valinamid;
- *N*-[(10H-Phenothiazin-2-yloxy)acetyl]glycyl-3-cyclohexyl-*N¹*-[(3S)-2-hydroxytetrahydrofuran-3-yl]-L-alaninamid;
- *N*-[(10H-Phenothiazin-2-yloxy)acetyl]glycyl-*N*-[(3S)-2-hydroxytetrahydrofuran-3-yl]-L-phenylalaninamid;
- *N*-[(10H-Phenothiazin-2-yloxy)acetyl]glycyl-*N¹*-[(3S)-2-hydroxytetrahydrofuran-3-yl]-*N²*-isobutylglycinamid;
- *N*-[2-Methyl-2-(10H-phenothiazin-2-yloxy)propanoyl]glycyl-*N¹*-[(3S)-2-methoxytetrahydrofuran-3-yl]-L-leucinamid;
- *N*-[2-Methyl-2-(10H-phenothiazin-2-yloxy)propanoyl]glycyl-*N¹*-[(3S)-2-hydroxytetrahydrofuran-3-yl]-L-leucinamid;
- *N*-(10,11-Dihydro-5H-dibenzo[b,f]azepin-3-ylcarbonyl)-L-leucyl-*N¹*-[(3S)-2-methoxytetrahydrofuran-3-yl]-L-leucinamid;
- *N*-(10,11-Dihydro-5H-dibenzo[b,f]azepin-3-ylcarbonyl)-L-leucyl-*N¹*-[(3S)-2-hydroxytetrahydrofuran-3-yl]-L-leucinamid;
- *N*-[(5-Acetyl-10,11-dihydro-5H-dibenzo[b,f]azepin-3-yl)carbonyl]-L-leucyl-*N¹*-[(3S)-2-methoxytetrahydrofuran-3-yl]-L-leucinamid;
- 2-Methyl-*N*-[(10H-phenothiazin-2-yloxy)acetyl]alanyl-*N¹*-[(3S)-2-hydroxytetrahydrofuran-3-yl]-L-leucinamidi
oder ein Salz einer dieser Verbindungen.

5. Eine Verbindung der allgemeinen Formel (I) nach Anspruch 1 oder ein pharmazeutisch annehmbares Salz einer solchen Verbindung als Medikament.

6. Pharmazeutische Zusammensetzung, die als Wirkstoff eine Verbindung der allgemeinen Formel (I) nach Anspruch 1 oder ein pharmazeutisch annehmbares Salz einer solchen Verbindung und mindestens einen pharmazeutisch annehmbaren Träger enthält.

7. Verwendung einer Verbindung der allgemeinen Formel (I) nach Anspruch 1 oder eines pharmazeutisch annehmbaren Salzes einer solchen Verbindung zur Herstellung eines Medikaments, das zur Hemmung der Calpaine bestimmt ist.

8. Verwendung einer Verbindung der allgemeinen Formel (I) nach Anspruch 1 oder eines pharmazeutisch annehmbaren Salzes einer solchen Verbindung zur Herstellung eines Medikaments, das zur Hemmung der Lipidperoxidation bestimmt ist.

9. Verwendung einer Verbindung der allgemeinen Formel (I) nach Anspruch 1 oder eines pharmazeutisch annehmbaren Salzes einer solchen Verbindung zur Herstellung eines Medikaments, das zur Hemmung der Calpaine und der Lipidperoxidation bestimmt ist.

10. Verwendung einer Verbindung der allgemeinen Formel (I) nach Anspruch 1 oder eines pharmazeutisch annehmbaren Salzes einer solchen Verbindung zur Herstellung eines Medikaments, das zur Behandlung von Störungen und Krankheiten bestimmt ist, die aus der Gruppe ausgewählt sind, die besteht aus den Entzündungs- und Immunkrankheiten, den kardiovaskulären und zerebrovaskulären Krankheiten, den Störungen des zentralen oder peripheren Nervensystems, der Osteoporose, den Muskeldystrophien, den proliferativen Krankheiten, Grauem Star, Abstoßungsreaktionen nach Organtransplantationen und Autoimmun- und Viruserkrankungen.
